# EUROPEAN PATENT APPLICATION

(11) **EP 1 875 899 A1**
(43) Date of publication of application: **09.01.2008**
(21) Application number: 06013496.2
(22) Date of filing: 29.06.2006
(51) Int. Cl.: A61K 31/00, A61K 31/415, A61K 31/4418, A61P 29/00

(54) **Use of 5HT7 receptor agonists for the treatment of pain**

(71) Applicant: LABORATORIOS DEL DR. ESTEVE, S.A., 08041 Barcelona (ES)
(72) Inventor: Vela Hernandez, Jose Miguel, 08041 Barcelona (ES); Torrens-Jover, Antonio, 08221 Tarressa (ES); Buschmann, Helmut H., 08960 Sant Just Desvem (ES); Romero-Alonso, Luz, 08041 Barcelona (ES)
(74) Representative: Peters, Hajo

(57) **Abstract**

The present invention refers to the use of 5HT7 receptor agonists for the treatment of the symptoms of pain, especially certain subtypes of pain like neuropathic pain, the prevention or the prophylaxis of the symptoms of pain, like neuropathic pain, especially certain subtypes of neuropathic pain or allodynia.

## Description

### Field of the invention

The present invention refers to the use of 5HT7 receptor agonists for the treatment of the symptoms of pain, especially certain subtypes of pain like neuropathic pain, the prevention or the prophylaxis of the symptoms of pain, like neuropathic pain, especially certain subtypes of neuropathic pain or allodynia.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210), Even though pain is always subjective its causes or syndromes can be classified.

Especially neuropathic pain which in the past years has developed into a major health problem in broad areas of the population needs a very specific treatment, especially considering that any treatment of neuropathic pain is extremely sensitive to the causes behind the pain, be it the disease ultimately causing it or the mechanistic pathway over which it develops.

Hyperalgesia and allodynia account for clinically relevant states such as inflammation and neuropathic pain. These conditions are characterized by alterations of pain perception that include enhanced sensitivity to noxious stimuli (hyperalgesia) and abnormal pain sensitivity to previously non-painful stimuli (allodynia). From the mechanistic point of view, the retationship between central sensitization and these pain associated behaviors, hyperalgesia and allodynia, has been recognized. Central sensitization is a term coined to describe the increased excitability of CNS nociceptive neurons triggered by persistent input or peripheral injury (Wooif, 1983). In fact, many popular models of post-injury hypersensitivity revolve around the notion of central sensitization whereby injury-evoked afferent discharges cause spinal cord neurons to reduce their stimulation threshold, to expand their receptive fields and to increase their responsiveness to different peripheral drives (Woolf, 1983; Hylden et al., 1989: Woolf and Thompson 1991; Coderre and Katz, 1997).

Subplantar capsaicin injection results in acute pain for 5 min followed by hyperalgesia/allodynia 15 min after the injection (Gilchrist et al., 1996). Immediate capsaicin-induced acute pain involves the activation of peripheral nociceptors as capsaicin acts primarily on transient receptor potential vanilloid 1 ion channel (TRPV1, formerly vanilloid receptor VR1) expressed by primary nociceptive afferents. In contrast, delayed hyperalgesia/allodynia involves the sensitization of spinal cord neurons (Baumann et al., 1991; LaMotte et al., 1992; Torebjörk et al., 1992). The release of glutamate and substance P and activation of their respective receptors in the spinal cord, key neurotransmitter systems implicated in spinal central sensitization (Woolf and Thompson, 1991; Khasabov et al., 2002), underlie mechanical allodynia secondary to subplantar capsaicin injection (Laird et al., 2001; Hwang et al., 2004; Soliman et al., 2005). On this basis, capsaicin sensitization represents a useful model for correlative behavioral, physiological and pharmacological studies in pain conditions involving central sensitization.

Many different neurotransmitter systems, ion channels and enzymes have been implicated in pain transmission, processing and controlling. Among them, serotonin (5-hydroxtryptamine [5-HT]), produced by central and peripheral serotonergic neurons but also by platelets and mast cells after tissue injury, has been described to exert algesic or analgesic effects depending on the site of action and the receptor subtype it acts on (Eide and Hole, 1993; Millan, 2002). At the peripheral level, after nerve injury, 5-HT is released in increased amounts and interacts with different 5-HT receptors present on C-fibers (Sommer, 2004). Acting in combination with other inflammatory mediators, 5-HT may ectopically excite and sensitize afferent nerve fibers, thus contributing to peripheral sensitization and hyperalgesia following inflammation and nerve injury (Beck and Handwerker, 1974: Obata et al., 2000). The central serotonin system has also been the subject of considerable research over the last twenty years. Both 5-HT and noradrenaline (NE)-mediated pathways of descending inhibition have been described extensively. These monoaminergic tracts arise from the midbrain and brainstem and terminate on the spinal cord to suppress sensory transmission and consequently produce analgesia. However, descending pathways projecting to the dorsal horn not only suppress (descending inhibition) but they may also potentiate (descending facilitation) nociceptive messages. In the case of the descending 5-HT system, both inhibition and facilitation have been described depending on the 5-HT receptor involved and on their divergent neuronal localization (Millan, 2002; Suzuki et al., 2004; Oyama et al., 1996).

Different drugs acting on the central 5-HT system have been investigated and/or used as analgesics. Among them, antidepressants are frequently used as adjuvants for analgesic treatment of pain. A significant amount of evidence supports the use of tricyclic antidepressants (TCAs) and other 5-HT and NE reuptake inhibitors (SNRIs) in the management of chronic pain, but because of their effects on multiple systems, they are associated with numerous undesirable side effects (Carter and Sullivan, 2002; Mattia et al., 2002: Barkin and Barkin, 2005). The newer selective 5-HT reuptake inhibitors (SSRIs), having only 5-HT-receptor-mediated side effects, have not been thoroughly studied but they seem to be less efficacious than TCAs and SNRIs in treating chronic pain (Barkin and Fawcett, 2000; Bomholt et al., 2005; Briley, 2004; Maizels and McCarberg, 2005; Sindrup et al., 2005; Stahl et al., 2005). In addition, available data suggest that increased 5-HT levels do not invariably inhibit nociceptive processing. Rather, it elicits a spectrum of pro- and antinociceptive actions that are receptor-dependent and a function of stimulus quality and modality (Millan, 2002; Suzuki et al., 2004).

As reviewed by Millan (2002), different 5-HT receptors have been implicated in analgesia. Among them, 5-HT_{1A}, 5-HT_{1H/1D}, 5-HT_{2A}. 5-HT_{2C} and 5-HT₃ have received most attention (Eide and Hole, 1993; Oyama et al., 1996; Obata et al., 2000; Kayser et al., 2002; Colpaert, 2006),

A recent Immunocytochemical study investigating 5-HT₇ receptor distribution at the lumbar level revealed that 5-MT₇ immunolabelling is localized mainly in the two superficial laminae of the dorsal horn and in small and medium-sized dorsal root ganglion cells, which is consistent with a predominant role in nociception (Doly et al., 2005). In rat and human dorsal root ganglia, the presence of 5-HT₇ receptor messenger RNA has also been detected (Pierce et al., 1996, 1997). Electron microscopic examination of the dorsal horn revealed three main localizations: 1) a postsynaptic localization on peptidergic cell bodies in laminae I-III and in numerous dendrites; 2) a presynaptic localization on unmyelinated (presumably of primary afferent origin) and thin myelinated peptidergic fibers (from intrinsic cells); and 3) a localization on astrocytes in lamina I and II (Doly et al., 2005). Regarding its function, a possible pronociceptive role of peripheral and spinal 5-HT₇ receptors has been described in the formalin test based on local paw and intrathecal administration of 5-HT or the non-selective 5-carboxamidotryptamine (5-CT) serotonergic agonist and reversion of their effects by the 5-HT₇ receptor antagonist SB-269970 (Rocha-González, 2005).

As especially neuropathic pain has developed into a major health problem in broad areas of the population and needs a very specific treatment, it was the underlying problem solved by this invention to find new ways of treating pain, especially neuropathic pain.

So, the main object of this invention is the use of a compound binding to the 5HT7 receptor and acting as an agonist in the production of a medicament for the treatment of pain.

This/these compound/s may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable ratio,

While working on compounds binding to the 5HT7 receptor and which act there as agonists it was surprisingly found out that these compounds could act on the treatment of pain, especially neuropathic pain, with a high potency.

"Treating" or "treatment" as used in this application are defined as including the treatment of the symptoms of pain, especially neuropathic pain, especially certain subtypes of neuropathic pain - as well as treatment of the disease or disease consequences causing the symptoms, the prevention or the prophylaxis of the symptoms of pain, especially neuropathic pain, especially certain subtypes of neuropathic pain - as well as the prevention or the prophylaxis of the disease or disease consequences causing the symptoms. Preferably "treating" or "treatment" as used in this application are defined as including the treatment of the symptoms of pain, especially neuropathic pain, especially certain subtypes of neuropathic pain - as well as treatment of the disease consequences causing the symptoms, the prevention or the prophylaxis of the symptoms of pain, especially neuropathic pain, especially certain subtypes of neuropathic pain - as well as the prevention or the prophylaxis of the disease consequences causing the symptoms. Most preferably "treating" or "treatment" as used in this application are defined as including the treatment of the symptoms of pain, especially of neuropathic pain, especially certain subtypes of neuropathic pain, and the prevention or the prophylaxis of the symptoms of pain, especially of neuropathic pain, especially certain subtypes of neuropathic pain.

The superfamily of serotonin receptors (5-HT) includes 7 classes (5-HT₁-5-HT₇) encompassing 14 human subclasses are described in many positions in literature especially in D. Hoyer, et al., Neuropharmacology, 1997, 36, 419. Thus:
"The 5HT7 receptor" as used in this application is well known and defined.
"The 5HT1A receptor" as used in this application is well known and defined.
All other "5HT receptors" as used in this application are well known and defined.

"Compound binding to the 5 HT7 receptor" as used in this application is/are defined as having a Kᵢ Value in their binding to the 5HT7-receptor ≤ 2 µM.

Assays that may be used for determining the affinity and selectivity of a 5-HT7 receptor agonist and/or other affinities to 5-HT receptors are well known in the art and especially measuring the affinities to these receptors are offered by service companies like CEREP (128, rue Danton, 92500 Rueil. Malmaison, France) or or MDS Pharma Services (Rosa de Lima 1bis, 2° 28290 Las Matas (Madrid), Spain). It is possible to classify a compound with 5-HT receptor affinity as agonist (also as inverse agonist or antagonist) according to the reference of S. M. Stahl, Essential Psychopharmacology, Neuroscientific basis and practical applications, Ed. Cambridge, 1996, Chapter 3. The respective part of the literature is hereby incorporated by reference and forms part of the disclosure.

An "Agonist" is defined as a compound that binds to a receptor and has an intrinsic effect, and thus, increases the basal activity of a receptor when it contacts the receptor. Full agonists show the maximum effect like in this case 5CT or AS-19 on the 5-HT7 receptor, whereas a partial agonist like MSD-5a is giving less (e.g. 80%) of the response of the full agonist as a maximum.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Some known subgroups of pain are acute pain, chronic pain, visceral pain and including also headaches, especially migraine. A subgroup of pain being of special interest in connection with this invention is neuropathic pain, which is the pain treated as highly preferred embodiment of the invention.

Therefore a preferred use according to the invention is the use of a compound binding to the 5HT7 receptor and acting as an agonist in the production of a medicament in which the pain is neuropathic pain, especially hyperalgesia or allodynia.

"Neuropathic pain" is defined by the IASP as "pain initiated or caused by a primary lesion or dysfunction in the nervous system" (IASP, Classification of chronic pain. 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system". One of the many reasons causing neuropathic pain is diabetes, especially diabetes II.

According to the IASP "allodynia" is defined as "a pain due to a stimulus which does not normally provoke pain" (IASP, Classification of chronic pain, 2nd Edition, IASP Press (2002). 210).

According to the IASP "hyperalgesia" is defined as "an increased response to a stimulus which is normally painful(IASP, Classification of chronic pain, 2nd Edition, IASP Press (2002), 211).

In a preferred embodiment of the invention the medicament is used for the treatment of neuropathic pain - or its subgroups - in which the stimulus evoking the neuropathic pain is mechanical.

In another embodiment of the invention the medicament is used for the treatment of neuropathic pain or its subgroups - in which the stimulus evoking the neuropathic pain is thermal.

In another preferred embodiment of the use according to the invention the neuropathic pain, is selected from central pain, hyperpathia, peripheral neuropathic pain or peripheral neurogenic pain, causalgia, hyperesthesia, neuralgia, neuritis, or neuropathy.

According to the IASP "central pain" is defined as "a pain initiated or caused by a primary lesion or dysfunction in the central nervous system" (IASP, Classification of chronic pain, 2nd Edition, IASP Press (2002), 211).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain. 2nd Edition, IASP Press (2002), 210).

According to the IASP "hyperesthesia" is defined as "increased sensitivity to stimulation, excluding the senses" (IASP, Classification of chronic pain, 2nd Edition, IASP Press (2002), 211).

According to the IASP "neuralgia" is defined as "Pain in the distribution of a nerve or nerves" (IASP, Classification of chronic pain, 2nd Edition, IASP Press (2002), 212).

According to the IASP "neuritis" is defined as "Inflammation of a nerve or nerves" (IASP, Classification of chronic pain, 2nd Edition, IASP Press (2002), 212).

According to the IASP "neuropathy" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition. IASP Press (2002), 212).

The IASP draws the following difference between "allodynia", "hyperalgesia" and "hyperpathia" (IASP, Classification of chronic pain, 2nd Edition. IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

Especially preferred embodiments of the invention encompass the use of a compound with a very specific binding to the 5HT7 receptor being in its binding profile more specific in its affinity (thus showing a lower Ki) to the 5HT7 receptor than in its affinity to other 5HT Receptors.

Therefore another preferred use according to the invention is the use of a compound binding to the 5HT7 receptor and acting as an agonist, wherein the compound binding to the 5-HT7 receptor is binding with a higher affinity - expressed as a lower Ki-value - to the 5HT7 receptor than to the 5-HT1A receptor, especially binding with an affinity higher by a factor of at least 10, preferably with an affinity higher by a factor of at least 30, more preferably with an affinity higher by a factor of at least 50, most preferably with an affinity higher by a factor of at least 100.

By way of exemplifying the above paragraph an compound X - specific to the 5HT7 receptor -, assumed to have an affinity - expressed as a Ki value- of 1 nM and an affinity to the 5HT1A receptor of a Ki value of 42 nM.

Another preferred use according to the invention is the use of a compound binding to the 5HT7 receptor and acting as an agonist, wherein the compound binding to the 5-HT7 receptor is binding with a higher affinity - expressed as a lower Ki-value - to the 5HT7 receptor than to the any other 5-HT receptor: especially binding with an affinity higher by a factor of at least 10, preferably with an affinity higher by a factor of at least 30, more preferably with an affinity higher by a factor of at least 50, most preferably with an affinity higher by a factor of at least 100.

Another preferred use according to the invention is the use of a compound binding to the 5HT7 receptor and acting as an agonist, wherein the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM.

Another preferred use according to the invention is the use of a compound binding to the 5HT7 receptor and acting as an agonist, wherein the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

Another preferred use according to the invention is the use of a compound binding to the 5HT7 receptor and acting as an agonist, wherein the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to any other 5HT receptor; especially binding with an affinity higher by a factor of at least 10, preferably with an affinity higher by a factor of at least 30, more preferably with an affinity higher by a factor of at least 50, most preferably with an affinity higher by a factor of at least 100,

Another preferred use according to the invention is the use of a compound binding to the 5HT7 receptor and acting as an agonist, wherein the compound is either a full or partial agonist.

In another preferred use according to the invention the compound used is either AS-19 or MSD5a, especially AS-19, optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate.

AS-1 9 [(2S)-(+)-8-(1,3,5-trimethylpyrazolin-4-yl)-2-(dimethylamino) tetralin] is defined by below sketched formula and is commercially available (e.g. through TOCRIS).

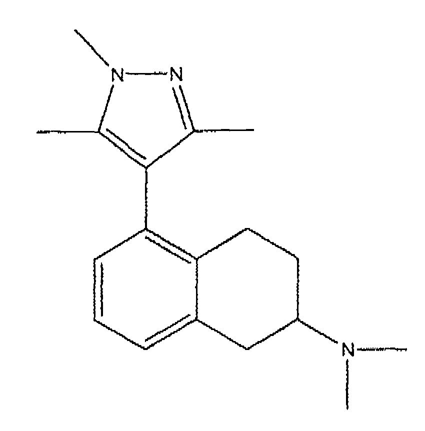

MSD-5a (corresponds to compound 5a in Thomson et al.. 2004; Merck Sharp & Dohme Research Laboratories) [2-(6-phenylpyridin-2-ylthio)-*N*,*N*-dimethyl ethanamine] is described in this literature including its synthesis.

Any use of a compound that is a prodrug of a compound used according to the invention - especially AS-19 and MSD-5a - is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Kragsgaard-Larsen et al. "Textbook of Drug design and Discovery" Taylor & Francis (April 2002).

Unless otherwise stated, the compounds used according to the invention especially AS-19 and MSD-5a - are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

In human therapeutics, the dose administered can be quite low depending on the route of administration. Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Any medicament according to the invention contains the active ingredient as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration can include intramuscular injection, intraveneous injection, subcutaneous injection, sublingual, bucal, patch through skin, oral ingestion, implantable osmotic pump, collagen implants, aerosols or suppository.

Included in this invention are especially also methods of treatments of a patient or a mammal, including men, suffering from pain, especially neuropathic pain using a compound binding to the 5HT7 receptor and acting as an agonist.

### Pharmacological methods:

### Method 1: Capsaicin-Test

### MATERIAL AND METHODS

### Animals

Male CD1 mice aged from 6 to 8 weeks old were used in these studies. Animals were housed in groups of five, provided with food and water ad libitum and kept in controlled laboratory conditions with the temperature maintained at 21 + 1 °C and light in 12 h cycles (on at 07:00 h and off at 19:00 h). Experiments were carried out in a soundproof and air-regulated experimental room. The number of mice ranged from 8 to 16 per group in each individual experiment.

### Drugs

Capsaicin (8-methyl-*N*-vanillyl 6-nonamide) was dissolved in 1 % DMSO (vehicle). Drugs used for treatments were: AS-19 [(2S)-(+)-8-(1,3,5-trimethylpyrazolin-4-yl)-2-(dimethylamino) tetralin], MSD-5a (corresponds to compound 5a in Thomson et al., 2004; Merck Sharp & Dohme Research Laboratories) [2-(6-phenylpyridin-2-ylthio)-*N,N*-dimethyl ethanamine], SB-258719 [(*R*)-3,*N*-Dimethyl-*N*-(1-methyl-,3-(4-methylpiperidin-1-yl) propyl) benzene sulfonamide], SB-269970 [(R)-3-(2-(2-(4-Methylpiperidin-1-yl) ethyl) pyrrolidine-1-sulfonyl) phenol]. WAY-100635 [(*N*-(2-(4-(2-methoxyphenyl)-1-piperazinyl) ethyl)-N-(2-pyridinyl) cyclohexane carboxamide] and F-13640 [(3-Chloro-4-fluoro-phenyl)-[4-fluoro-4-{[(5-methyl-pyridin-2-ylmethyl) amino]-methyl}piperidin-1-yl] methanone]. AS-19 was dissolved in 1% DMSO and the rest of compounds were prepared as aqueous solutions, Doses were calculated based on the molecular weight of the free base. All the compounds and vehicles (1% DMSO or physiological saline) were administered in a volume of 5 ml/kg through the subcutaneous (s.c.) route. When two compounds were co-administered, they were injected s.c. in opposite flanks of the mice.

### Selectivity of drugs

There is a remarkable need of selective 5-HT₇ ligands, particularly agonists. This has hindered pharmacological studies exploring the role played by this receptor. In this study, pharmacological evidence is presented to prove the involvement of the 5-HT₇ receptor in the control of allodynia following capsaicin sensitization. Information on the selectivity of the pharmacological tools used is thus a key issue and has been compiled in this section.

AS-19 is a potent 5-HT₇ receptor agonist (IC₅₀ = 0.83 nM; Tocris) (Sanin et al., 2004). There was a lack of information in the literature regarding its affinity for receptors other than 5-HT₇ (Perez-Garcia and Meneses (2005). Hence, the affinity of AS-19 for a total of 70 receptors, transporters or ion channels was evaluated in a commercial screening package (MDS Pharma Services) and the binding profile was presented in Table 1. As shown, its affinity for human 5-HT receptors is 5-HT₇ (Kᵢ = 0.605 nM) > 5-HT_{1A} (Kᵢ = 89.7 nM) > 5-HT_{5A} (Kᵢ = 98.5 nM) and it has no significant affinity for other 5-HT receptor subtypes or other receptors, transporters or ion channels included in the screening package.
MSD-5a is also an agonist showing high affinity for 5-HT₇ (Kᵢ = 0.6 nM), but its selectivity against 5-HT_{1A} (Kᵢ = 16 nM) is not as high as in the case of AS-19 (Table 1). In addition, it has partial agonist activity, giving 80% of the response of the full agonist 5-CT in 5-HT₇ receptors (Thomson et al., 2004).

SB-258719 was identified as the first selective 5-HT₇ receptor antagonist, with 100-fold selectivity over a range of other receptors (Forbes et al., 1998). SB-269970 was also identified as a potent and selective 5-HT₇ receptor antagonist (Lovell et al., 2000). Particularly noteworthy is the lack of affinity at the 5-HT_{1A} receptor by both 5-HT₇ receptor antagonists used in this study (Forbes et al., 1998; Lovell et al., 2000). Regarding functionality, SB-258719 acts as a pure antagonist whereas inverse agonist activity has been described for SB-269970 (Mahé et al., 2004).

### Capsaicin model of neurogenic pain

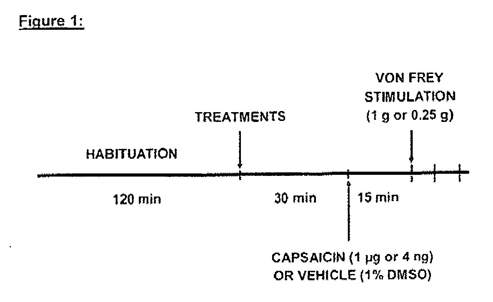

Subplantar capsaicin injection initially evokes a nocifensive behavior that is characterized by lifting and guarding the injected paw and typically lasts up to 5 min following injection. Afterwards, hypersensitivity to both thermal and mechanical stimuli is evidenced (Gilchrist et al., 1996). In this study, sensitization by subplantar capsaicin injection was used to assess the effect of several 5-HT, ligands on mechanical allodynia.
Mice were injected with capsaicin (1*µ*g or 4 ng in 20*µ*l of 1% DMSO) or 1% DMSO (vehicle) into the mid-plantar surface of the right hind paw. Drug treatments with 5-HT₇ ligands were done 30 min before capsaicin injection and withdrawal latencies to a mechanical stimulus were determined 15 min after capsaicin injection (Fig. 1).

### Evaluation of mechanical allodynia: von Frey test

Mechanical allodynia was quantified by measuring the hind paw withdrawal response latency to von Frey filament stimulation. Mice were placed into compartment enclosures in a test chamber with a framed metal mesh floor and allowed to acclimate to their environment for 120 min before testing. During this time the animal initially demonstrated exploratory behavior, but subsequently stopped exploring and stood quietly with only occasional bouts of grooming. The mechanical stimulus was applied to the plantar surface of the right, capsaicin-injected hind paw from below the mesh floor by using an automated testing device (dynamic plantar aesthesiometer; Ugo Basile, Italy) that lifts a straight filament (0.5 mm diameter). The filament exerted a fixed upward pressure (1 g or 0.25 g) on the plantar surface. When the animal withdrew its hind paw, the mechanical stimulus automatically stopped and the latency time was recorded. Latency was defined as the time from the onset of exposure to the filament to the cessation of the pressure when the sensor detected the withdrawal of the paw. A cut-off latency of 60 sec was imposed. Paw withdrawal latencies were measured in triplicate for each animal.

### Experimental approaches

When fixed at 1 g, the pressure exerted by the filament is insufficient to induce timely pain responses in normal control mice (in the absence of capsaicin) and thus prompt withdrawal responses found 15 min after sensitization with 1 µg capsaicin reflect mechanical allodynia. This approach enabled us to evaluate the possible antiallodynic effect of 5-HT₇ ligands.
Alternatively, in some experiments, only 4 ng (instead of 1*µ*g) of capsaicin were injected into the paw and the pressure exerted by the filament was reduced to 0.25 g (instead of 1 g). Mice receiving this minor amount of capsaicin (250 times less than those used to induce allodynia) did not display allodynia when mechanically stimulated with a low upward pressure of 0.25 g. This approach was used to test the possibility that treatments could exert a proallodynic effect. The Figure 1 summarizes the two different experimental approaches carried out in this study.

### Data analysis

Data are presented as mean paw withdrawal latency responses in sec ± S.E.M. Capsaicin-injectcd hind paw withdrawal responses to mechanical stimulation of treated mice were compared with those of untreated (vehicle-treated) mice. Statistical analysis to test intergroup comparisons was made using an initial one-way analysis of variance followed by the Newman-Keuls multiple comparison test. The level of significance was set at p < 0.05.

### REFERENCES

Barkin RL. Fawcett J. (2000) The management challenges of chronic pain: the role of antidepressants. Am J Ther, 7:31-47.
Barkin RL, Barkin S. (2005) The role of venlafaxine and duloxetine in the treatment of depression with decremental changes in somatic symptoms of pain, chronic pain, and the pharmacokinetics and clinical considerations of duloxetine pharmacotherapy. Am J Ther, 12:431-438.
Baumann TK. Simone DA. Shain CN, LaMotte RH (1991) Neurogenic hyperalgesia: the search for the primary cutaneous afferent fibers that contribute to capsaicin-induced pain and hyperalgesia. J Neurophysiol, 66:212-227.
Beck PW, Handwerker HO. (1974) Bradykinin and serotonin effects on various types of cutaneous nerve fibers. Pflugers Arch, 347:209-222.
Bomholt SF, Mikkelsen JD, Blackburn-Munro G. (2005) Antinociceptive effects of the antidepressants amitriptyline, duloxetine, mirtazapine and citalopram in animal models of acute, persistent and neuropathic pain. Neuropharmacology, 48:252-263.
Briley M. (2004) Clinical experience with dual action antidepressants in different chronic pain syndromes. Hum Psychopharmacol. Suppl 1:S21-25.
Carter GT, Sullivan MD. (2002) Antidepressants in pain management. Curr Opin Investig Drugs, 3:454-458.
Coderre TJ, Katz J. (1997) Peripheral and central hyperexcitability: differential signs and symptoms in persistent pain. Behav Brain Sci, 20:404-419.
Colpaert FC. Tarayre JP. Koek W. Pauwels PJ. Bardin L, Xu XJ, Wiesenfeld-Hallin Z, Cosi C. Carilla-Durand E., Assie MB, Vacher B. (2002) Large-amplitude 5-HT1A receptor activation: a new mechanism of profound, central analgesia. Neuropharmacology, 43:945-958.
Colpaert FC. (2006) 5-HT1A receptor activation: new molecular and neuroadaptive mechanisms of pain relief. Curr Opin Investig Drugs. 7:40-47.
Doly S. Fischer J, Brisorgueil MJ, Verge D, Conrath M. (2005) Pre- and postsynaptic localization of the 5-HT7 receptor in rat dorsal spinal cord: immunocytochemical evidence. J Comp Neurol, 490:256-269.
Eide PK, Hole K. (1993) The role of 5-hydroxytryptamine (5-HT) receptor subtypes and plasticity in the 5-HT systems in the regulation of nociceptive sensitivity. Cephalalgia, 13:75-85.
Forbes IT, Dabbs S, Duckworth DM, Jennings AJ. King FD, Lovell PJ, Brown AM. Collin L, Hagan JJ, Middlemiss DN. Riley GJ, Thomas DR, Upton N, (1998) (R)-3,N-dimethyl-N-[1-methyl-3-(4-methyl-piperidin-1-yl) propyl]benzenesulfonamide: the first selective 5-HT7 receptor antagonist. J Med Chem, 41:655-657.
Forster EA, Cliffe IA, Bill DJ, Dover GM, Jones D, Reilly Y, Fletcher A. (1995) A pharmacological profile of the selective silent 5-HT1A receptor antagonist, WAY-100635. Eur J Pharmacol, 281:81-88.
Gilchrist HD, Allard BL. Simone DA (1996) Enhanced withdrawal responses to heat and mechanical stimuli following intraplantar injection of capsaicin in rats. Pain, 67:179-188.
Harte SE, Kender RG, Borszcz GS. (2005) Activation of 5-HT1A and 5-HT7 receptors in the parafascicular nucleus suppresses the affective reaction of rats to noxious stimulation. Pain, 113:405-415.
Hwang SJ, Burette A. Rostioni A, Valtschanoff JG. (2004) Vanilloid receptor VR1-positive primary afferents are glutamatergic and contact spinal neurons that co-express neurokinin receptor NK1 and glutamate receptors. J Neurocytol, 33:321-339.
Hylden JL, Nahin RL, Traub RJ, Dubner R. (1989) Expansion of receptive fields of spinal lamina I projection neurons in rats with unilateral adjuvant-induced inflammation: the contribution of dorsal horn mechanisms. Pain, 37:229-243.
Kayser V, Aubel B, Hamon M, Bourgoin S. (2002) The antimigraine 5-HT1B/1D receptor agonists, sumatriptan, zolmitriptan and dihydroergotamine, attenuate pain-related behaviour in a rat model of trigeminal neuropathic pain. Br J Pharmacol, 137:1287-1297.
Khasabov SG, Rogers SD, Ghilardi JR, Peters CM, Mantyh PW. Simone DA. (2002) Spinal neurons that possess the substance P receptor are required for the development of central sensitization. J Neurosci, 22:9086-9098.
Khawaja X, Evans N, Reilly Y, Ennis C, Minchin MC. (1995) Characterisation of the binding of [3H]WAY-100635, a novel 5-hydroxytryptamine1A receptor antagonist, to rat brain. J Neurochem, 64:2716-2726.
Laird JM, Roza C, De Felipe C, Hunt SP, Cervero F. (2001) Role of central and peripheral tachykinin NK1 receptors in capsaicin-induced pain and hyperalgesia in mice. Pain, 90:97-103.
LaMotte RH, Lundberg LE, Torebjörk HE (1992) Pain, hyperalgesia and activity in nociceptive C units in humans after intradermal injection of capsaicin. J Physiol (Lond), 448:749-764.
Lovell PJ, Bromidge SM, Dabbs S, Duckworth DM, Forbes IT, Jennings AJ, King FD, Middlemiss DN, Rahman SK, Saunders DV, Collin LL, Hagan JJ. Riley GJ, Thomas DR. (2000) A novel, potent, and selective 5-HT7 antagonist: (R)-3-(2-(2-(4-methylpiperidin-1-yl)ethyl)pyrrolidine-1-sulfonyl) phenol (SB-269970). J Med Chem. 2000, 43:342-345.
Lovenberg TW, Baron BM, de Lecea L, Miller JD, Prosser RA, Rea MA, Foye PE, Racke M. Slone AL, Siegel BW, Danielson PE. Sutcliffe JG, Erlander MG (1993) A novel adenylyl cyclase-activating serotonin receptor (5-HT7) implicated in the regulation of mammalian circadian rhythms, Neuron, 11:449-458,
Mahé C. Loetscher E. Feuerbach D. Muller W, Seiler MP. Schoeffter P. (2004) Differential inverse agonist efficacies of SB-258719, SB-258741 and SB-269970 at human recombinant serotonin 5-HT7 receptors. Eur J Pharmacol. 495:97-102.
Maizels M. McCarberg B. (2005) Antidepressants and antiepileptic drugs for chronic non-cancer pain. Am Fam Physician, 71:483-490.
Mattia C, Paoletti F, Coluzzi F. Boanelli A. (2002) New antidepressants in the treatment of neuropathic pain. A review. Minerva Anestesiol, 68:105-114.
Millan MJ. (2002) Descending control of pain. Prog Neurobiol. 66:355-474.
Obata H, Saito S, Ishizaki K, Goto F. (2000) Antinociception in rat by sarpogrelate, a selective 5-HT2A receptor antagonist, is peripheral. Eur J Pharmacol, 404:95-102.
Oyama T, Ueda M, Kuraishi Y, Akaike A. Satoh M. (1996) Dual effect of serotonin on formalin-induced nociception in the rat spinal cord. Neurosci Res, 25:129-135.
Perez-Garcia GS, Meneses A. (2005) Effects of the potential 5-HT7 receptor agonist AS 19 in an autoshaping learning task. Behav Brain Res, 163:136-140.
Perrone R, Berardi F, Colabufo NA, Lacivita E, Leopoldo M, Tortorella V. (2003) Synthesis and structure-affinity relationships of 1-[omega-(4-arly-1-piperazinyl)alkyl]1-aryl ketones as 5-HT7 receptor ligands. J Med Chem, 46:646-649.
Pierce PA, Xie GX, Levine JD, Peroutka SJ. (1996) 5-Hydroxytryptamine receptor subtype messenger RNAs in rat peripheral sensory and sympathetic ganglia: a polymerase chain reaction study. Neuroscience, 70:553-559.
Pierce PA, Xie GX, Meuser T, Peroutka SJ. (1997) 5-Hydroxytryptamine receptor subtype messenger RNAs in human dorsal root ganglia: a polymerase chain reaction study. Neuroscience. 81:813-819.
Rocha-González Hl, Meneses A, Carlton SM, Granados-Soto V. (2005) Pronociceptive role of peripheral and spinal 5-HT7 receptors in the formalin test. Pain, 117:182-192.
Reissig CJ, Eckler JR, Rabin RA, Winter JC. (2005) The 5-HT1A receptor and the stimulus effects of LSD in the rat. Psychopharmacology (Berl), 182:197-204.
Sanin A, Brisander M, Rosqvist S, Mohell N, Malberg A, Johansson A. (2004) 5-Aryl substituted (S)-2-(dimethylamino)-tetralins novel serotonin 5HT7 receptor ligands. In: Proceedings of the 14th Camerino-Noord Symposium. Ongoing Progress in the Receptor Chemistry, p.27.
Sleight AJ, Carolo C, Petit N, Zwingelstein C, Bourson A. (1995) Identification of 5-hydroxytryptamine 7 receptor binding sites in rat hypothalamus: sensitivity to chronic antidepressant treatment. Mol Pharmacol, 47:99-103.
Stahl SM, Grady MM, Moret C, Briley M. (2005) SNRIs: their pharmacology, clinical efficacy, and tolerability in comparison with other classes of antidepressants. CNS Spectr. 10(9):732-747
Suzuki R, Rygh LJ, Dickenson AH. (2004) Bad news from the brain: descending 5-HT pathways that control spinal pain processing. Trends Pharmacol Sci, 25-613-617.
Soliman AC, Yu JS, Coderre TJ. (2005) mGlu and NMDA receptor contributions to capsaicin-induced thermal and mechanical hypersensitivity. Neuropharmacology, 48:325-332.
Sindrup SH, Otto M, Finnerup NB, Jensen TS. (2005) Antidepressants in the treatment of neuropathic pain. Basic Clin Pharmacol Toxicol, 96:399-409.
Sommer C. (2004) Serotonin in pain and analgesia: actions in the periphery, Mol Neurobiol, 30:117-125.
Thomson CG, Beer MS, Curtis NR, Diggle HJ. Handford E, Kulagowski JJ. (2004) Thiazoles and thiopyridines: novel series of high affinity h5HT7 ligands. Bioorg Med Chem Lett, 14:677-680.
Torebjörk HE, Lundberg LE, LaMotte RH. (1992) Central changes in processing of mechanoreceptive input in capsaicin-induced secondary hyperalgesia in humans. J Physiol (Lond), 448;765-780.
Wilcox RE, Ragan JE, Pearlman RS, Brusniak MY. Eglen RM, Bonhaus DW, Tenner TE Jr, Miller JD. (2001) High-affinity interactions of ligands at recombinant guinea pig 5HT7 receptors. J Comput Aided Mol Des, 15:883-909.
Woolf CJ. (1983) Evidence for a central component of post-injury pain hypersensitivity- Nature, 306:686-688.
Woolf CJ, Thompson SW. (1991) The induction and maintenance of central sensitization is dependent on N-methyl-D-aspirtic acid receptor activation; implications for the treatment of post-injury pain hypersensitivity states. Pain, 44:293-299.
You HJ, Colpaert FC, Arendt-Nielsen L. (2005) The novel analgesic and high-efficacy 5-HT1A receptor agonist F 13640 inhibits nociceptive responses, wind-up, and after-discharges in spinal neurons and withdrawal reflexes. Exp Neurol, 191:174-183.

### Method 2: Partial sciatic nerve ligation

### INTRODUCTION

Neuropathic pain refers to pain as a result of damage (due to injury or disease) to the nervous system including nerves, spinal cord and certain CNS regions (Woolf and Mannion, 1999; Zimmermann, 2001). Patients with neuropathic pain often suffer from spontaneous pain, allodynia (pain response to normally innocuous stimuli) and hyperalgesia (exaggerated pain evoked by noxious stimuli). Direct evaluation of spontaneous pain in animals is not possible, although some signs such as excessive grooming, changes in exploratory behavior or weight bearing have been suggested as indications of spontaneous pain. In contrast, evoked pain (allodynia and hyperalgesia) to thermal or mechanical stimuli can be clearly observed in most animal models of neuropathic pain. From the mechanistic point of view, neuropathic pain-associated behaviors, allodynia and hyperalgesia, revolve around the notion of central sensitization, whereby injury-evoked increased and persistent afferent discharges cause spinal cord neurons to reduce their stimulation threshold, to expand their receptive fields and to increase their responsiveness to different peripheral drives (Woolf, 1983; Hylden et al., 1989; Woolf and Thompson 1991; Coderre and Katz, 1997).

Most neuropathic pain models are made by producing diseases or causing injuries to peripheral nerves. These methods to induce neuropathic pain differ in the location and type of peripheral nerve injury. The latter includes transection, loose or tight ligation, crush, perineural inflammation, tumor invasion, infection and toxic affectation (Wang and Wang, 2003). In an attempt to simulate causalgia as a result of partial nerve injury in humans, Seltzer el al. (1990) developed a rat model of neuropathic pain involving the ligation of 1/3 to 1/2 of the thickness of the sciatic nerve. Partial sciatic nerve ligated rats exhibit marked allodynia to von Frey hair stimulation and hyperalgesia to both thermal and mechanical noxious stimuli in the ipsilateral nerve-ligated paw. The evoked pain appears within hours after ligation and last for over 7 months. Similar results were obtained in mice by Malmberg and Basbaum (1998), who established some additional behavioral and neuroanatomical correlates and pointed up the reliability and objectivity of the partial sciatic nerve ligation model of neuropathic pain in mouse.

### MATERIAL AND METHODS

### Animals

Mate CD1 mice aged from 6 to 8 weeks old were used in these studies. Animals were housed in groups of five, provided with food and water ad libitum and kept in controlled laboratory conditions with the temperature maintained at 21 ± 1°C and light in 12 h cycles (on at 07:00 h and off at 19;00 h). Experiments were carried out in a soundproof and air-regulated experimental room. The number of mice ranged from 8 to 16 per group in each individual experiment.

### Drugs

Drugs used were: AS-19 [(2*S*)-(+)-8-(1,3,5-trimethylpyrazolin-4-yl)-2-(dimethylamino) tetralin] and SB-258719 [(*R*)-3,*N*-Dimethyl-*N*-(1-methyl-3-(4-methylpiperidin-1-yl) propyl) benzene sulfonamide]. AS-19 was dissolved in 1% DMSO and SB-258719 was prepared as aqueous solutions. Doses were calculated based on the molecular weight of the free base. Both compounds and vehicles (1 % DMSO or physiological saline) were administered in a volume of 5 ml/kg through the subcutaneous (s.c.) route. When compounds were co-administered, they were injected s.c. in opposite flanks of the mice.

### Selectivity of drugs

There is a remarkable need of selective 5-HT₇ ligands, particularly agonists. This has hindered pharmacological studies exploring the role played by this receptor. In this study, pharmacological evidence is presented to prove the involvement of the 5-HT₇ receptor in the control of allodynia and hyperalgesia in neuropathic pain conditions, information on the selectivity of the pharmacological tools used is thus a key issue and has been compiled in this section.
AS-19 is a potent 5-HT₇ receptor agonist (IC₅₀ = 0.83 nM: Tocris) (Sanin et al., 2004). Unfortunately, as pointed up by Perez-Garcia and Meneses (2005), there is a lack of information in the literature regarding its affinity for receptors other than 5-HT₇. Hence, the affinity of AS-19 for a total of 70 receptors transporters or ion channels was evaluated in a commercial screening package (MDS Pharma Services) and its binding profile presented in Table 1. As shown, AS-19 is over 100-fold selective for 5-HT₇. Its affinity for human 5-HT receptors is 5-HT, (Kᵢ= 0.605 nM) > 5-HT_{1A} (Kᵢ = 89.7 nM) > 5-HT_{5A} (Kᵢ = 98.5 nM) and it has no significant affinity for other 5-HT receptor subtypes or other receptors, transporters or ion channels included in the screening package.
SB-258719 was identified as the first selective 5-HT₇ receptor antagonist, with 100-fold selectivity over a range of other receptors (Forbes et al., 1998). Particularly noteworthy is its lack of affinity at the 5-HT_{IA} receptor. Regarding functionality, SB-258719 seems to act as a neutral antagonist (Mahé et al., 2004).

### Neuropathic pain model: partial sciatic nerve ligation

The partial sciatic nerve ligation model was used to induce neuropathic pain, according to the method previously described (Seltzer et al., 1990; Malmberg and Basbaum, 1998). This model consists of partial injury to the sciatic nerve at mid-thigh level. All surgical procedures for nerve injury were performed under isoflurane (IsoFlo^{®}, Abbott-Laborstorios Dr. Esteve, S.A., Barcelona, Spain) anesthesia (induction: 3%; surgery: 2%). Briefly, mice were anaesthetized and the common sciatic nerve was exposed at the level of the mid-thigh of the right hind paw. Partial nerve injury was produced at about 1 cm proximal to the nerve trifurcation by tying a tight ligature around approximately 33 to 50% the diameter of the sciatic nerve using 9-0 non-absorbable virgin silk suture (Alcon surgical, Texas, USA). Ligature enclosed the outer 1/3-1/2 sciatic nerve whereas the rest of the nerve (inner 2/3-1/2) was leaved "uninjured". The muscle was then stitched with 6-0 silk suture and the skin incision was closed with wound clips. Control, sham-operated mice underwent the same surgical procedure and the sciatic nerve was exposed, but not ligated. After surgery, animals were allowed to recover for 5 days prior to initiation of nociceptive behavioral tests (see experimental protocol below).

### Nociceptive behavioral tests

Allodynia to mechanical stimulus and hyperalgesia to noxious thermal stimulus were used as outcome measures of neuropathic pain and as indicators of the possible antinociceptive effect of drug treatments.

### Evaluation of mechanical allodynia: von Frey test

Mechanical allodynia was quantified by measuring the tactile threshold for hind paw withdrawal following von Frey filament stimulation. The day of the test, mice were placed into compartment enclosures in a test chamber with a framed metal mesh floor and allowed to acclimate to their environment for 1 h before testing. The mechanical stimulus was applied to the plantar surface of the right hind paw from below the mesh floor by using an automated testing device (dynamic plantar aesthesiometer; Ugo Basile, Italy) that lifts a straight filament (0.5 mm diameter). The filament exerted an increasing upward pressure on the plantar surface. The pressure increased linearly with time (0.1g/s; from 0 to 5 g in 50 s) until the mouse withdrew its paw. When the animal withdrew its hind paw, the mechanical stimulus automatically stopped and the force was recorded as the mechanical threshold. Thus, the mechanical threshold was defined as the pressure at which the sensor detected the withdrawal of the paw. A cut-off threshold of 5 g (50 s) was imposed. Paw withdrawal latencies were measured in triplicate for each animal.

### Evaluation of thermal hyperalgesia: plantar test

Thermal hyperalgesia was assessed using the plantar test by determination of the withdrawal latencies to a thermal stimulus following a modification of the test described by Hargreaves et al, (1988). The day of the test, mice were placed in a plantar test device (Plantar test, Ugo Basile, Italy) and allowed to acclimate to their environment for 1 h before testing. The plantar test device consisted of a plastic cubicle with a glass floor through which a mobile infrared photobeam was applied onto the plantar surface of the right hind paw. Latency time to withdrawal from the thermal stimulus was determined. The intensity of the infrared photobeam was adjusted to produce mean response latencies in control untreated mice of approximately 8 to 12 seconds. The response latency was determined using a timer linked to the photodiode motion sensors in the plantar test device. Response latency was defined as the time from the onset of exposure to the infrared photobeam to the cessation of the photobeam when the photodiode motion sensor detected the withdrawal of the paw of the mouse. Paw withdrawal latencies were measured in triplicate for each animal and the mean value was used for analysis. A cut-off latency of 20 seconds was imposed to avoid tissue damage.

### Experimental protocol

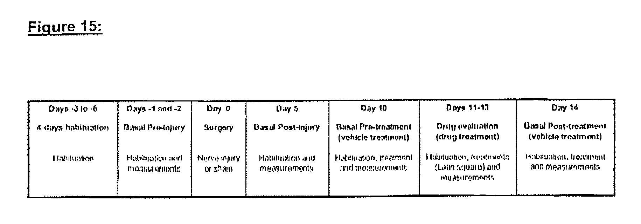

The effectiveness of acute treatments with 5-HT₇ ligands compared to vehicle on the expression of neuropathic pain was investigated based on the following experimental protocol. Mice were habituated for 1 h to the environment of the different experimental tests (either von Frey or plantar test, depending on the experiment) during 4 days. After the habituation period, pre-injury baseline responses in each test were established during 2 consecutive days. One day after baseline measurements, nerve injury or sham operation was induced as previously described. Mice were then tested in either the von Frey or the plantar test on day 5 after the surgical procedure to monitor post-injury responses. Post-injury baseline responses of mice treated with vehicle were obtained on day 10 (basal pre-treatment). On days 11-13 mice received treatment with three different doses of 5-HT₇ ligands following a *Latin square* design. Finally, on day 14 after surgery, baseline responses in vehicle-treated mice (basal post-treatment) were evaluated as an internal control to ensure that mechanical allodynia and thermal hyperalgesia were not influenced by previous treatments in the *Latin square* design. Behavioral evaluation was always done 30 min after treatment with either vehicle or 5-HT, ligands. Figure 15 summarizes the chronology of the experimental protocol.

### Data analysis

Data were primarily presented as pressure (grams) required for reaching the threshold of response (paw withdrawal) in the von Frey test or as latency (seconds) of paw withdrawal in the plantar test. In some cases, data were presented as percentages respect to pre-treatment baseline values. Values were always mean ± S.E.M.
Data obtained in the von Frey and plantar tests in treated mice were compared with those of untreated (vehicle-treated) mice. Statistical analysis to test intergroup comparisons was made using an initial one-way analysis of variance followed by the post hoc Newman-Keuls multiple comparison test. Differences were considered significant if the probability of error was less than 5% (the level of significance was set at p < 0.05).

The examples and figures in the following section describing pharmacological trials are merely illustrative and the invention cannot be considered in any way as being restricted to these applications.

### Figures:

### Figure 1) Scheme summarizing experimental protocols

Mice were habituated for 120 min and received s.c. treatment with 5-HT₇ ligands or vehicle 30 min before subplantar injection of capsaicin or vehicle. Paw withdrawal latencies to a mechanical stimulus (von Frey stimulation) were determined 15 min after subplantar capsaicin or vehicle injection. To test the possible antiallodynic effect of drugs, prompt allodynic responses were induced by injecting 1*µ*g of capsaicin followed by stimulation with 1 g of pressure. In contrast, to detect possible proallodynic effects of drugs, a low ineffective dose of 4 ng of capsaicin and a low subthreshold mechanical pressure of 0.25 g were used.

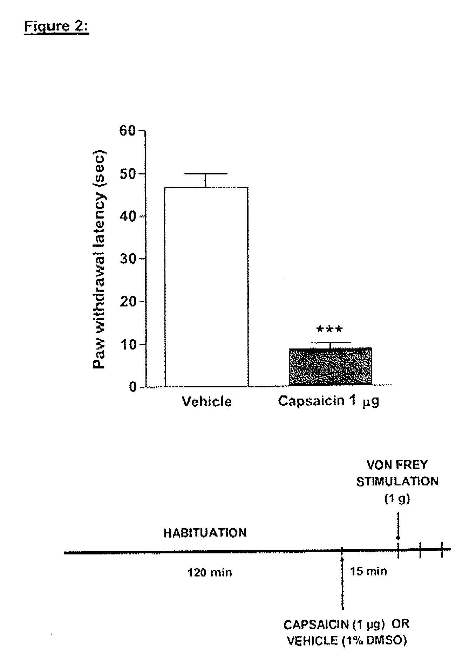

### Figure 2) Effect of subplantar capsaicin injection on the paw withdrawal latency to mechanical stimulation: mechanical allodynia

Figure 2 refers to example 1a). Mice receiving subplantar injection of 1 µg capsaicin developed mechanical allodynia, as evidenced by their significantly reduced paw withdrawal latencies to stimulation with a 1 gram-pressure filament when compared to mice subplantarly injected with vehicle (1% DMSO). In addition to the graph summarizing the results, the figure shows the scheme of the experimental protocol. *** p<0.001 vs. vehicle.

### Figure 3) Dose-response effect of AS-19 on mechanical allodynia induced by capsaicin

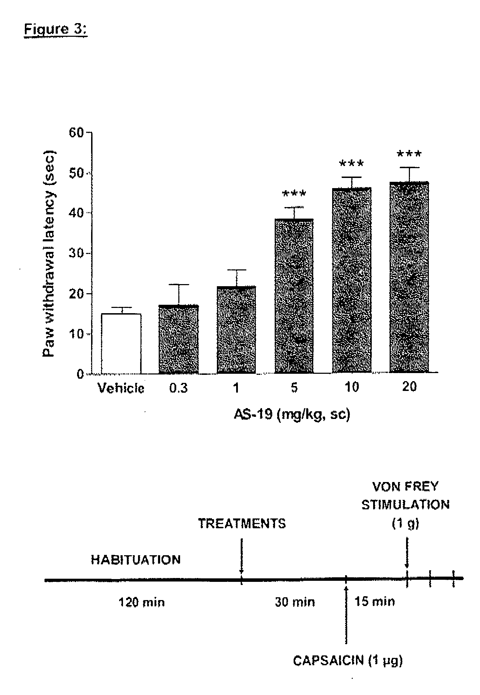

Figure 3 refers to Example 1b). Mechanical allodynia in mice receiving subplantar injection of 1 *µ*g capsaicin was dose dependently inhibited by treating mice with AS-19. In addition to the graph summarizing the results, the figure shows the scheme of the experimental protocol. *** p<0.001 vs. vehicle,

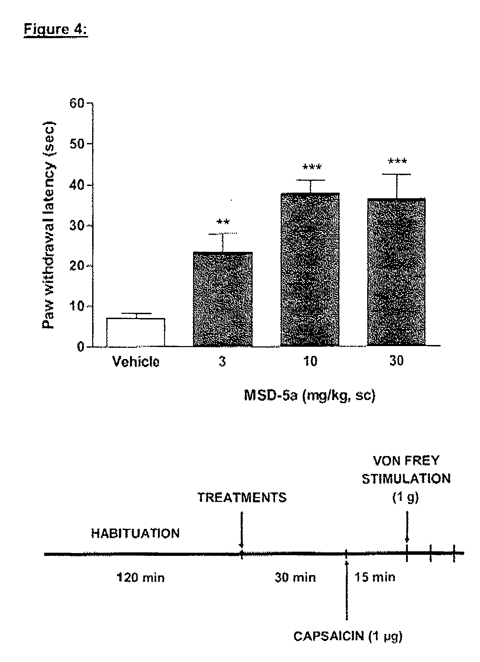

### Figure 4) Dose-response effect of MSD-5a on mechanical allodynia induced by capsaicin

Figure 4 refers to Example 1b). Mechanical allodynia in mice receiving subplantar injection of 1 *µ*g capsaicin was inhibited by treating mice with MSD-5a. In addition to the graph summarizing the results, the figure shows the scheme of the experimental protocol, ** p<0.01; *** p<0.001 vs. vehicle.

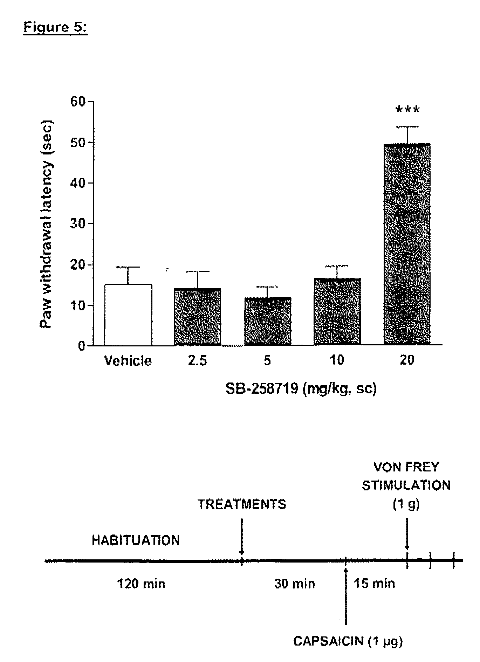

### Figure 5) Doso-response effect of SB-258719 on mechanical allodynia induced by capsaicin

Figure 5 refers to Example 1c). Mechanical allodynia in mice receiving subplantar injection of 1 *µ*g capsaicin was unaffected by treating mice with SB-258719 at. 2.5, 5 and 10 mg/kg. In contrast, treatment with SB-258719 at 20 mg/kg significantly increased paw withdrawal latencies respect to vehicle-treated mice. In addition to the graph summarizing the results, the figure shows the scheme of the experimental protocol. *** p<0.001 vs vehicle.

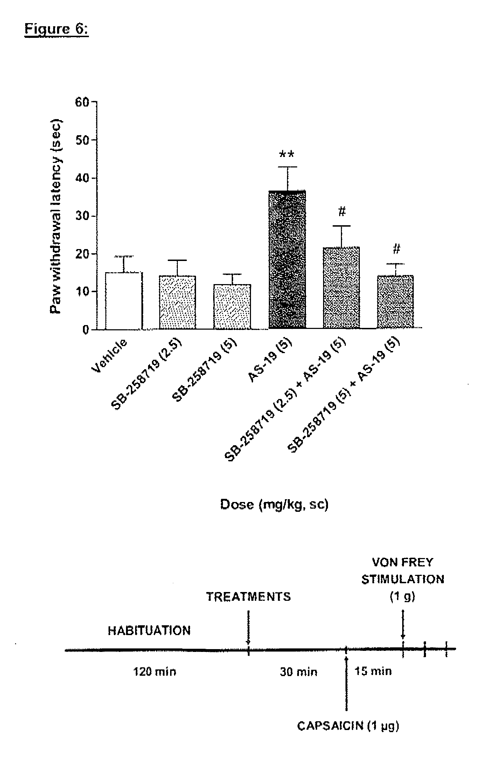

### Figure 6) Dose-dependent reversion of the antiallodynic effect of AS-19 by SB-258719

Figure 6 refers to Example 1c). Mechanical allodynia in mice receiving subplantar injection of 1 µg capsaicin was unaffected by treating mice with SB-258719 at 2.5 and 5 mg/kg, but these doses of SB-258719 were able to reverse the antiallodynic effect of 5 mg/kg of AS-19. In addition to the graph summarizing the results, the figure shows the scheme of the experimental protocol. ** p<0.01 vs. vehicle; # p<0.05 *vs.* AS-19 (5).

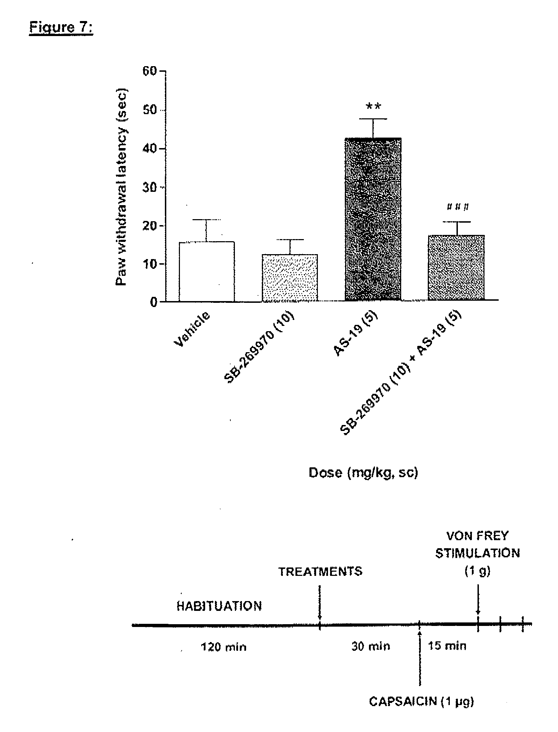

### Figure 7) Reversion of the antiallodynic effect of AS-19 by SB-269970

Figure 7 refers to Example 1c). Mechanical allodynia in mice receiving subplantar injection of 1 µg capsaicin was unaffected by the 5-HT, antagonist SB-269970 (10 mg/kg) but, when co-administered with AS-19 (5 mg/kg), it blocked the antiallodynic effect of the agonist. In addition to the graph summarizing the results, the figure shows the scheme of the experimental protocol, ** p<0.01 vs. vehicle: # # # p<0.001 vs. AS-19(5).

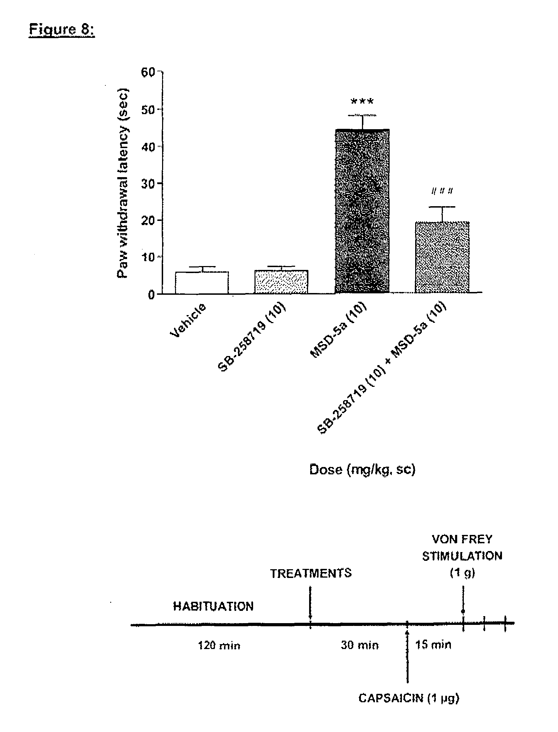

### Figure 8) Reversion of the antiallodynic effect of MSD-5a by SB-258719

Figure 8 refers to Example 1c). Mechanical allodynia in mice receiving subplantar injection of 1 µg capsaicin was inhibited by MSD-5a (10 mg/kg) and SB-258719 (10 mg/kg) blocked this antiallodynic effect. In addition to the graph summarizing the results, the figure shows the scheme of the experimental protocol. *** p<0.001 vs. vehicle; # # # p<0.001 vs. MSD-5a (10).

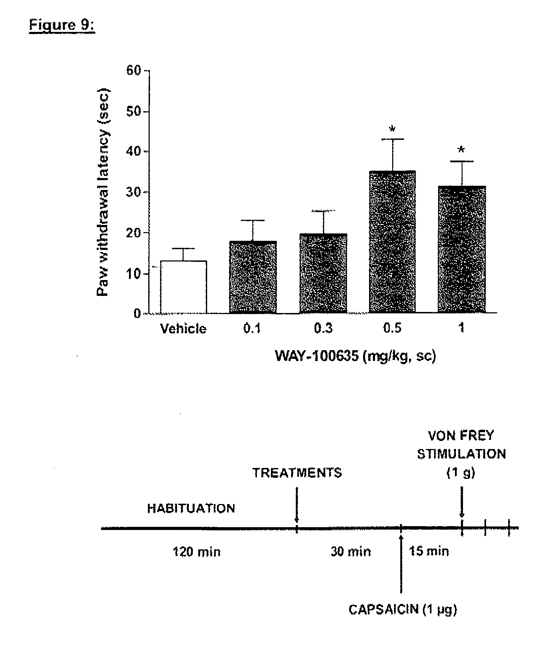

### Figure 9) Dose-response effect of WAY-100535 on mechanical allodynia induced by capsaicin

Figure 9 refers to Example 1c). Mechanical allodynia in mice receiving subplantar injection of 1 µg capsaicin was unaffected by treating mice with WAY-100635 at 0.1 and 0.3 mg/kg, but not at 0.5 and 1 mg/kg, doses at which a significant increase in paw withdrawal latency was observed. In addition to the graph summarizing the results, the figure shows the scheme of the experimental protocol. * p<0,05 vs vehicle.

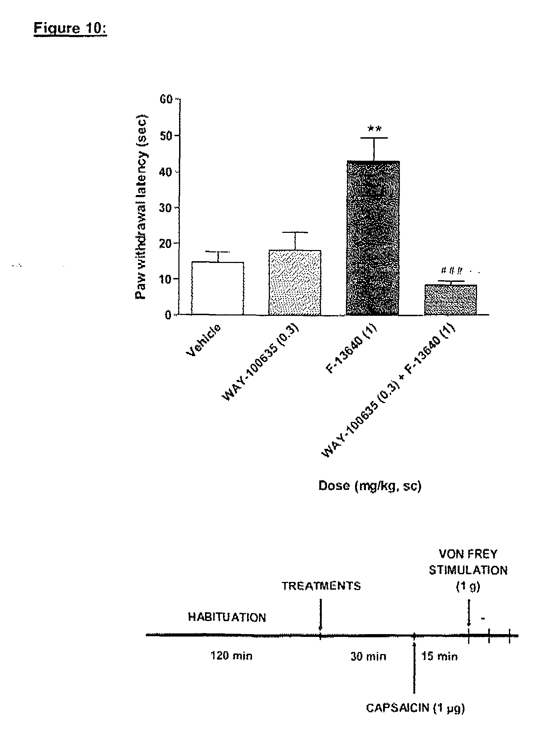

### Figure 10) Reversion of the antiallodynic effect of F-13640 by WAY-100635

Figure 10 refers to Example 1c). Mechanical allodynia in mice receiving subplantar injection of 1 µg capsaicin was inhibited by F-13640 (1 mg/kg) and WAY-100635 (0,3 mg/kg) blocked this antiallodynic effect. In addition to the graph summarizing the results, the figure shows the scheme of the experimental protocol. ** p<0.01 vs. vehicle; # # # p<0.001 vs. F-13640 (1).

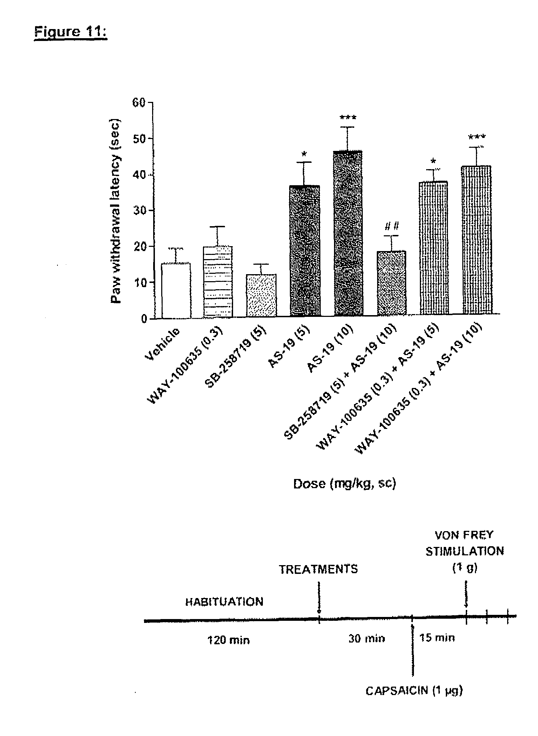

### Figure 11) Reversion of the antiallodynic effect of AS-19 by SB-258719 or WAY-100635

Figure 11 refers to Example 1c). Mechanical allodynia in mice receiving subplantar injection of 1 *µ*g capsaicin was unaffected after treatment with SB-258719 (5 mg/kg) or WAY-100635 (0.3 mg/kg). However, SB-258719 but not WAY-100635 significantly inhibited the antiallodynic effect of AS-19. In addition to the graph summarizing the results, the figure shows the scheme of the experimental protocol. * p<0.05: ** p<0.01 vs. vehicle; ## p<0.01 vs. AS-19 (10).

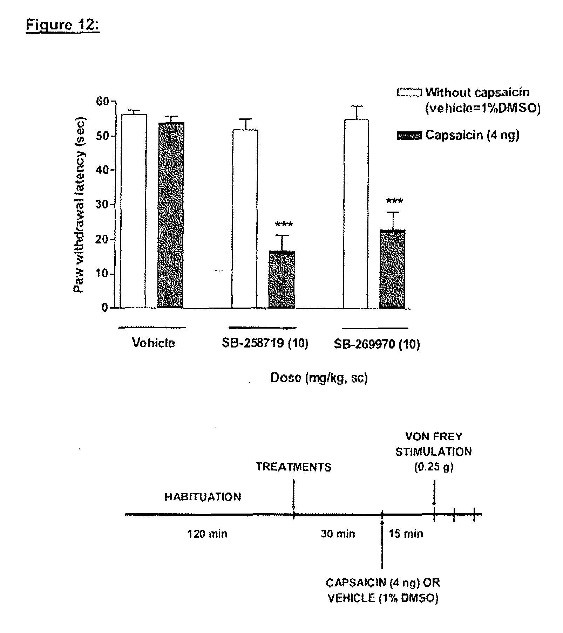

### Figure 12) Potentiation of capsaicin-induced mechanical allodynia by SB-258729 and SB-269970.

Figure 12 refers to Example 1d). In the absence of capsaicin or when a minor dose of 4 ng was injected into the paw, mice did not exhibit allodynia when mechanically stimulated with a low upward pressure of 0,25 g. However, allodynia was evidenced in mice receiving 4 ng of capsaicin when treated with the 5-HT₇ antagonists SB-258719 or SB-269970 at 10 mg/Kg, showing a proallodynic effect of 5-HT₇ receptor antagonists. In addition to the graph summarizing the results, the figure shows the scheme of the experimental protocol. *** p<0.001 vs. "without capsaicin".

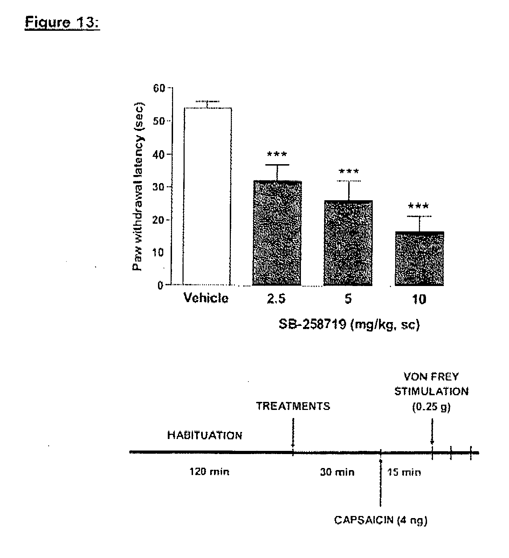

### Figure 13) Dose-dependent potentiation of capsaicin-induced mechanical allodynia by SB-258729.

Figure 13 refers to Example 1d), SB-258719 dose-dependently promoted allodynia in mice subptantarly injected with a minor dose of 4 ng and mechanically stimulated with a low upward pressure of 0.25 g. In addition to the graph summarizing the results, the figure shows the scheme of the experimental protocol. *** p<0.001 vs. vehicle.

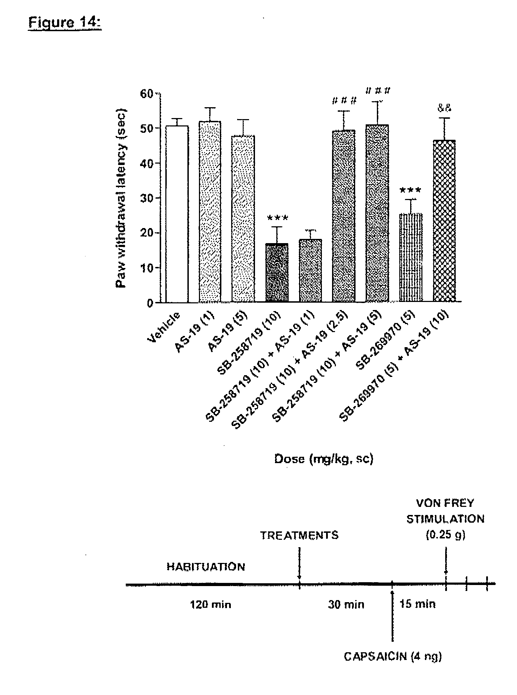

### Figure 14) Reversion of the proallodynic effect of SB-258719 and SB-269970 by AS-19

Figure 14 refers to Example 1d). AS-19 had no effect in non-allodynic conditions after subplantar injection of 4 ng of capsaicin and low pressure (0.25 g) stimulation, but it was able to reverse the proallodynic effect of SB-258719 (10 mg/Kg) and SB-269970 (5 mg/Kg). In addition to the graph summarizing the results, the figure shows the scheme of the experimental protocol. *** p<0.001 vs, vehicle; # # # p<0.001 vs, SB-258719 (10); && p<0.01 vs. SB-269970 (5)

### Figure 15: Schematic Treatment in Sciatic Nerve Ligation:

Mice were habituated during 4 days and basal pre-injury values were obtained during the next 2 consecutive days by means of the von Frey or the plantar test. The following day mice were subjected to surgery and 5 days later post-injury baseline values were obtained in the mentioned tests. On day 10 post-injury mice received vehicle and measurements were done to obtain pre-treatment basal values. Measurements in drug-treated mice were done on days 11-13 post-injury following a *Latin square* design. Finally, post-treatment basal values were obtained on day 14. Habituation of mice during the first 4 days and every time before measurements lasted 1 h. When treated, measurements were always done 30 min after administration of the vehicle or the tested compound.

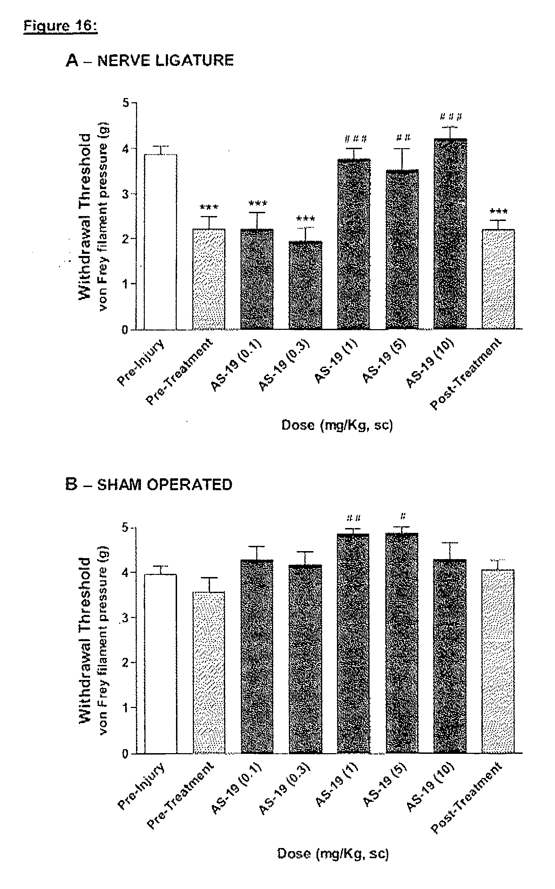

### Figure 16: Dose-response effect of AS-19 on mechanical allodynia

Figure 16 refers to example 3a). Pressure threshold for evoking ipsilateral hind paw withdrawal to von Frey filament stimulation was evaluated in nerve-injured (A) and sham-operated (B) mice before surgery (pre-injury), on day 10 post-injury after treatment with vehicle (pre-treatment), on days 11-13 post-injury after treatment with different doses of AS-19 and finally on day 14 post-injury after treatment with vehicle (post-treatment). Note that mechanical allodynia develops after partial sciatic nerve ligation (but not after sham operation) and that AS-19 at doses of 1-10 mg/kg exerted antiallodynic effects. *** p<0.001 vs Pre-Injury; " p<0,05, " " p<0.01, " " " p<0.001 vs Pre- and Post-Treatment (Newman-Keuls Multiple Comparison Test posf-ANOVA).

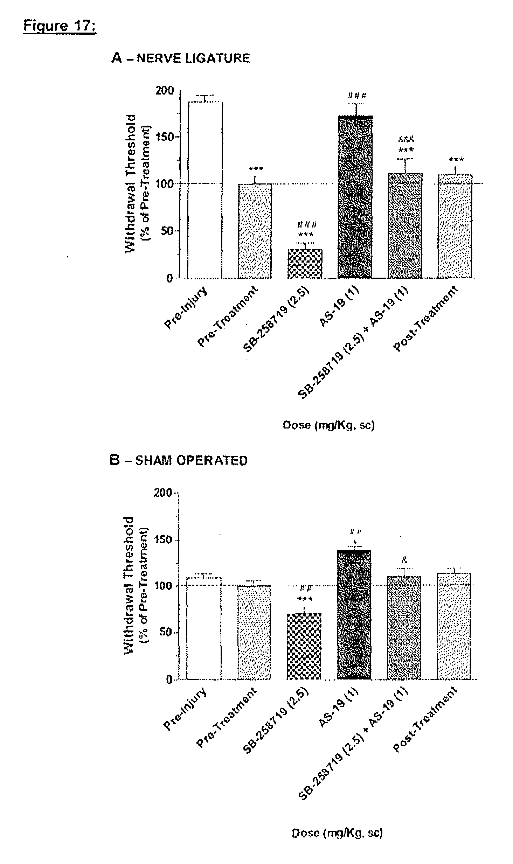

### Figure 17: Reversion of the effects of AS-19 and SB-258719 on mechanical allodynia

Figure 17 refers to example 3a). Pressure threshold for evoking ipsilateral hind paw withdrawal to von Frey filament stimulation was evaluated in nerve-injured (A) and sham-operated (B) mice before surgery (pre-injury), on day 10 post-injury after treatment with vehicle (pre-treatment), on day 11-13 post-injury after treatment with SB-258719, AS-19 or their combination, and finally on day 14 post-injury after treatment with vehicle (post-treatment). Data were presented as percentages respect to pre-treatment values. Note that SB-258719 (2.5 mg/kg) exerted proallodynic AS-19 (1 mg/kg) antiallodynic effects and that co-administration of both compounds blocked their respective effects both in nerve-injured and sham-operated mice. *p<0.05, *** p<0,001 vs Pre-Injury; ^{##} p<0.01, ^{###} p<0.001 vs Pre- and Post-Treatment; ^{&}p<0.05. ^{&&&} p<0.001 vs AS-19 (10) and SB-258719 (2.5) (Newman-Keuls Multiple Comparison Test post-ANOVA).

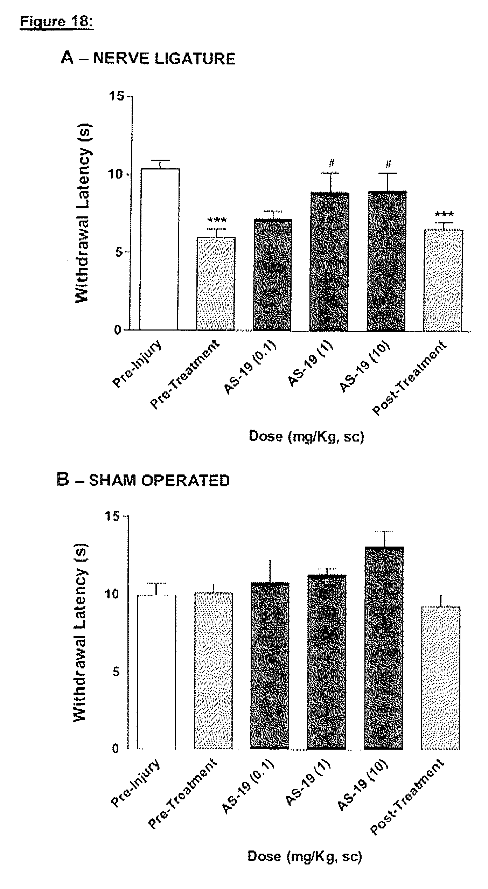

### Figure 18: Dose-response effect of AS-19 on thermal hyperalgesia

Figure 18 refers to example 3b), Latency of ipsilateral hind paw withdrawal to thermal stimulus was evaluated in nerve-injured (A) and sham-operated (B) mice before surgery (pre-injury), on day 10 post-injury after treatment with vehicle (pre-treatment), on days 11-13 post-injury after treatment with different doses of AS-19 and finally on day 14 post-injury after treatment with vehicle (post-treatment). Note that thermal hyperalgesia develops after partial sciatic nerve ligation (but not after sham operation) and that AS-19 at doses of 1 and 10 mg/kg exerted antihyperalgesic effects.*** p<0.01 vs Pre-Injury; "p<0.05 vs Pre- and Post-Treatment (Newman-Keuls Multiple Comparison Test post-ANOVA).

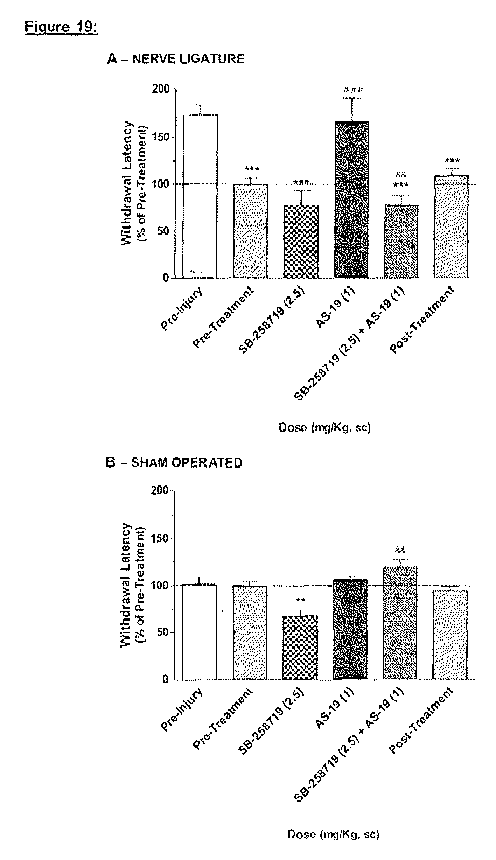

### Figure 19: Reversion of the effects of AS-19 and SB-258719 on thermal hyperalgesia

Figure 19 refers to example 3b), Latency of ipsilateral hind paw withdrawal to thermal stimulus was evaluated in nerve-injured (A) and sham-operated (B) mice before surgery (pre-injury), on day 10 post-injury after treatment with vehicle (pre-treatment), on day 11-13 post-injury after treatment with SB-258719. AS-19 or their combination, and finally on day 14 post-injury after treatment with vehicle (post-treatment). Data were presented as percentages respect to pre-treatment values. Note that the analgesic effect of AS-19 (1 mg/kg) in nerve-injured mice and the hyperalgesic effect of SB-258719 (2.5 mg/kg) in sham-operated mice were blocked when both compounds were co-administered. ** p<0.01, *** p<0.001 vs Pre-injury-, ^{###} p<0.001 vs Pre- and Post-Treatment; && p<0.01 vs AS-19 (10) in A and SB-258719 (2.5) in B (Newman-Keuls Multiple Comparison Test post-ANOVA).

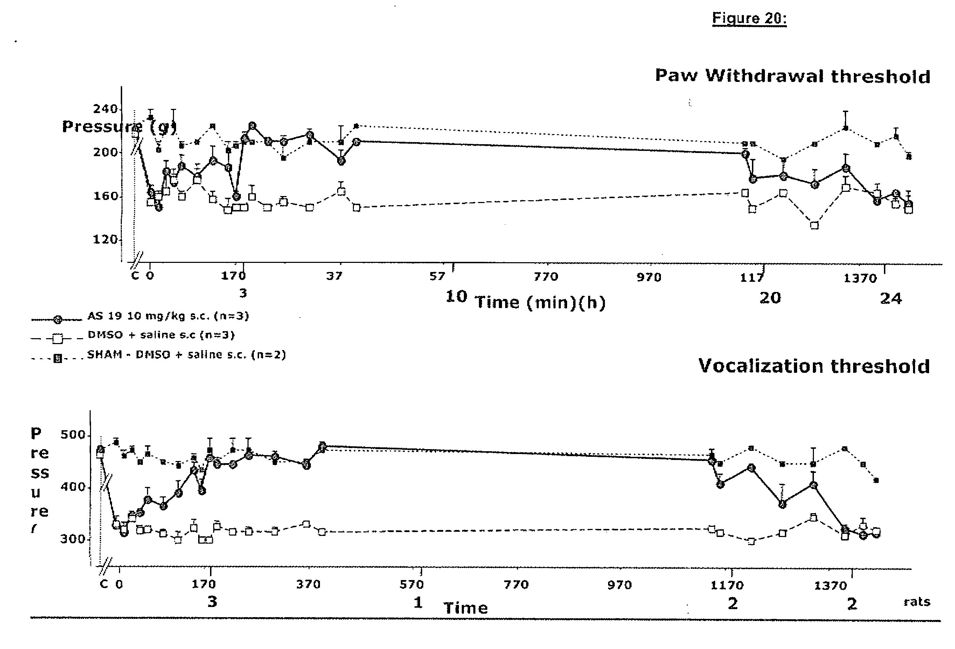

### Fig. 20 : Effect of AS-19 after sciatic nerve ligation

This figure refers to example 4 and shows the antinociceptive effect of AS-19.

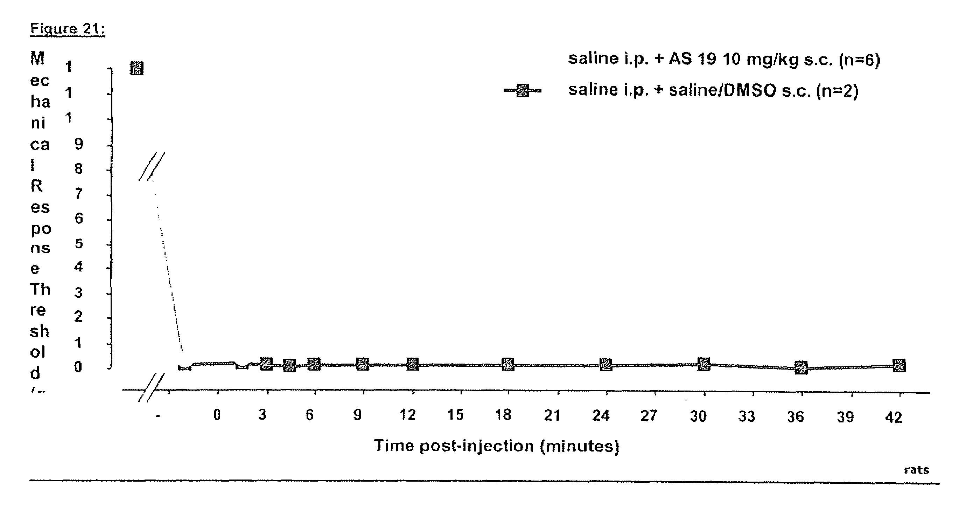

### Fig. 21: Effect of AS-19 after infraorbital nerve ligation

This figure refers to example 5 and shows the antinociceptive effect of AS-19.

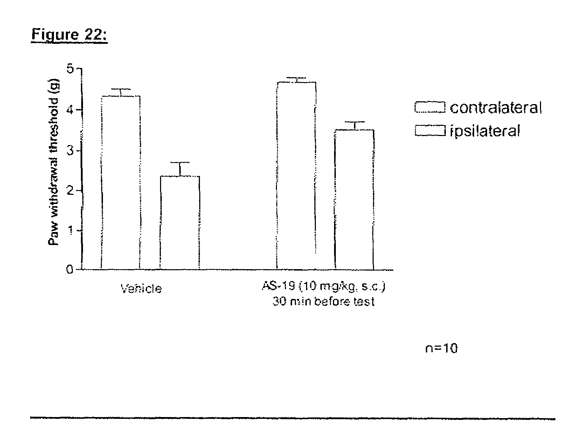

### Fig. 22: Effect of AS-19 after carageenan injection: mechanical allodynia

As can be seen by Figure 22, AS-1 9 has an antiallodynic effect in acute inflammation.

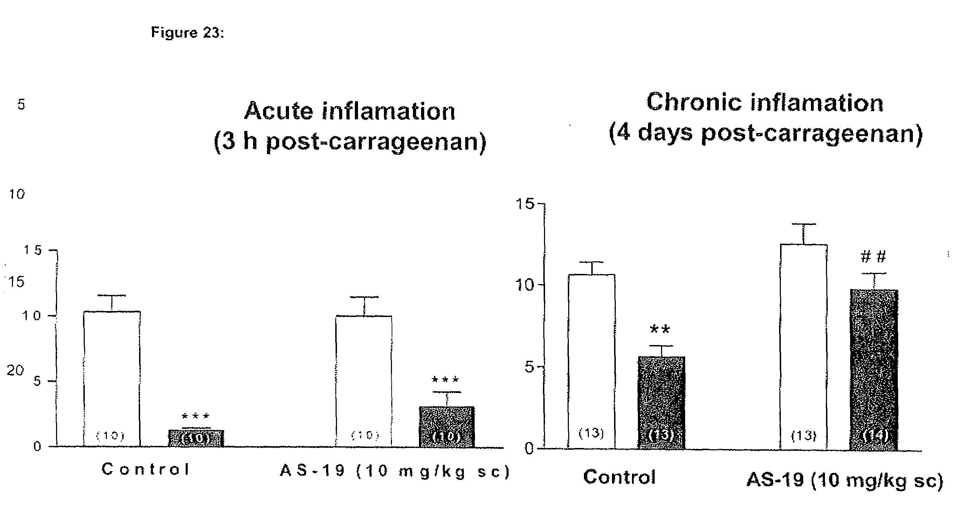

### Fig. 23: Effect of AS-19 after carageenan injection: theramal hyperalgesia

As can be seen by Figure 23, AS-19 has an antihyperalgesic effect in acute and especially chronic inflammation.

### Examples:

### Example 1: Capsaicine-Test:

### a) Capsaicin sensitization.

Capsaicin sensitization produces mechanical allodynia. Mice injected with capsaicin (1*µ*g) into the mid-plantar surface of the right hind paw developed mechanical allodynia 15 min after the injection. The pressure exerted by the filament (1 g) was below the threshold of feeling of paws from control mice (control withdrawal latencies of mice injected with 1% DMSO were close to the 60 sec cut-off). However, 15 min after subplantar capsaicin injection, the same "innocuous", "non-noxious" pressure (1 g) was perceived as painful and the withdrawal latency was severely reduced (Fig. 2). This abnormal pain sensitivity to previously non-painful mechanical stimuli is known as mechanical allodynia and in our model results from capsaicin sensitization.

### b) Selective 5-HT₇ receptor agonists dose-dependently inhibit capsaicin-induced mechanical allodynia

Systemic subcutaneous administration of the 5-HT₇ receptor agonist AS-19 dose-dependently inhibited capsaicin-induced mechanical allodynia (Fig. 3). Significantly increased paw withdrawal latencies of mice treated with AS-19 were found at the doses of 5, 10 and 20 mg/Kg when comparing to mice treated with vehicle. The antiallodynic effect reached the maximum at the dose of 10 mg/kg.
Similar to AS-19, MSD-5a, another 5-HT₇ receptor agonist used in this study, exerted antiallodynic effects (Fig. 4). At doses of 3. 10 and 30 mg/kg, administration of MSD-5a to mice receiving capsaicin increased the latencies of nociceptive paw withdrawal responses to mechanical stimulation compared to vehicle-treated mice. Differences were significant (p>0.05) at all three tested doses and reached the maximum at 10 mg/kg.

### c) Reversion of the antiallodynic effect of 5-NT₇ receptor agonists by selective 5-HT₇ receptor antagonists

In order to confirm that activation of 5-HT₇ receptors is unambiguously responsible for the inhibition of capsaicin-induced mechanical allodynia exerted by AS-19 and MSD-5a, we used two different 5-HT₇ receptor antagonists (SB-258719 and SB-269970) to try to pharmacologically reverse the antiallodynic effect of agonists.
First we performed a dose-response study of the effect of SB-258719 by itself on capsaicin-induced mechanical allodynia (Fig. 5). SB-258719 was s.c injected at doses of 2.5, 5, 10 and 20 mg/kg and no effects were observed except at the highest dose, at which an antiallodynic effect was evidenced.
In the next experiment, low doses of SB-258719 having no antiallodynic effects were chosen to try to reverse the antiallodynic effect exerted by 5 mg/kg of AS-19. As shown in Figure 6, SB-258719 at 2.5 and 5 mg/kg had no effect whereas treatment with AS-19 (5 mg/kg) increased paw withdrawal latencies. Interestingly, the analgesic (antiallodynic) effect of AS-19 was dose-dependently inhibited when co-administered with SB-258719.
Pharmacological reversion of the antiallodynic effect of AS-19 was also successfully accomplished by using another 5HT₇ receptor antagonist, SB-269970, at a dose (10 mg/kg) that by itself did not modify paw withdrawal latencies after subplantar capsaicin injection (Fig. 7). Finally, we also blocked the antiallodynic effect produced by the other 5HT₇ receptor agonist, MSD-5a (10 mg/kg), using the antagonist SB-258719 (5 mg/kg) (Fig. 8). Altogether, preceding data using two different 5HT₇ receptor agonists and confirmation by pharmacological reversion with two different 5HT₇ receptor antagonists, strongly suggest that activation of 5HT₇ receptors exerts an antiallodynic effect after capsaicin sensitization.
As AS-19 has some affinity for 5-HT_{1A} receptors in addition to bind to 5HT₇ receptors (see *Selectivity of drugs* in the Methods section; Table 1) and as 5-HT_{1A} receptor agonists are known to exert analgesic effects in a number of animal models (Colpaert, 2006), we next evaluated the possibility that actions on 5-HT_{1A} receptors could also contribute to the antiallodynic effects of AS-19. To accomplish that, AS-19 was administered together with the selective 5-HT_{1A} antagonist WAY-100635 (Foster et al., 1995; Khawaja et al., 1996) at 0.3 mg/kg. a dose that had no effect on capsaicin-induced mechanical allodynia (Fig. 9) but largely blocks the effect of the selective 5-HT_{1A} agonist F-13640 (Colpaert et al., 2002) (Fig. 10). Similarly, this dose has been shown to block the 5-HT_{1A}-mediated effects induced by 8-OH-DPAT (ID₅₀ = 0.01 mg/kg) (Foster et al., 1995) and other 5-HT_{1A} agonists (Reissig et al., 2005; You et al., 2005). Intriguingly, the antiallodynic effect of AS-19 at 5 and 10 mg/kg was unaffected in the presence of WAY-100635 but was abolished by SB-258719 (Fig. 11), indicating that activation of 5-HT₇ but not of 5-HT_{1A} receptors underlies the antiallodynic effect exerted by AS-19.

### d) Selective 5-HT₇ receptor antagonists exert proallodynic effects

Activation of 5-HT₇ receptors by selective agonists has an antiallodynic effect in mice sensitized with capsaicin, but do selective 5-HT₇ receptor antagonists exert a proallodynic effect? To investigate this possibility we reduced both the dose of capsaicin (from 1 *µ*g to 4 ng) and the upward pressure exerted by the filament (from 1 g to 0.25 g). Under these conditions, mice did not display mechanical allodynia. However, when treated with SB-258719 or SB-269970, mice receiving this minor amount of capsaicin (4 ng) and subjected to this low-pressure stimulation (0.25 g) showed a marked allodynia (Fig. 12). Interestingly, the proallodynic effect of 5-HT₇ receptor antagonists was observed in mice injected with 4 ng of capsaicin but not in mice injected with vehicle (1% DMSO), suggesting that a minimal sensitizing challenge is needed for the antagonists to exert their effect.
The proallodynic effect of SB-258719 was dose-dependent (Fig. 13) and was inhibited by AS-19 (Fig. 14). Promotion of allodynia by the other antagonist, SB-269970, was also inhibited by AS-19 (Fig. 14), pointing to the involvement of 5-HT₇ receptors in such an effect.

### CONCLUSIONS

5-HT₇ receptors play a key role in the control of mechanical allodynia elicited by capsaicin sensitization:
- **Activation of 5HT₇ receptors by selective agonists dose-dependently inhibits capsaicin-induced mechanical allodynia.**
   The antiallodynic effect was evidenced by using two different selective 5HT₇ receptor agonists (AS-19 and MSD-5a). AS-19 and MSD-5a blocked this behavioral manifestation of pain and restored the nociceptive response of capsaicin-sensitized mice to baseline values found in control unaffected mice.
- **In contrast, blocking of 5HT₇ receptors by selective antagonists produces a dose-dependent proallodynic effect.**
   The proallodynic effect was evidenced by using two different selective 5HT₇ receptor antagonists (SB-258719 and SB-289970).
- **Antiallodynic effects by agonists and proallodynic effects by antagonists can be reversed by antagonists and agonists, respectively.**
   The analgesic (antiallodynic) effects of AS-19 and MSD-5a were both inhibited by using two different selective 5HT₇ receptor antagonists (SB-258719 and SB-289970). In the same way, promotion of allodynia by the antagonists SB-258719 and SB-269970 was blocked by the AS-19 agonist.

**These results reveal the involvement of the 5-HT₇ receptor in the control of pain in conditions involving central sensitization and point to a new potential therapeutic use of 5-HT₇ receptor agonists as analgesics.**

### Example 2:

### Affinities to selected 5HT-Receptors of compounds used.

The binding data collected in the table 1 below were either obtained from Literature (Thomson et al, (2004), Forbes et al. (1998) and Lovell et al. (2000)), obtained by MDS Pharma Services, or done by the applicant, using well documented standard procedures.

**Table 1. Receptor affinity profiles in radioligand binding assays**

| **Receptor** | **Affinity [kᵢ (nM)]** | | | |
|---|---|---|---|---|
| | **AS-19** | **MSD-5a** | **SB-258719** | **SB-269970** |
| h5-HT_{1A} | 89.7 | 16 | n.s | n.s. |
| h5-HT_{1B} | -- | -- | n.s. | 1000 |
| h5-HT_{1D} | -- | -- | n.s. | n.s. |
| h5-HT_{1E} | -- | -- | n.s. | n.s. |
| h5-HT_{1F} | -- | -- | n.s | n.s |
| r5-HT₁ non-selective | 48.1 | -- | -- | -- |
| h5-HT_{2A} | -- | 320 | n.s. | n.s. |
| h5-HT_{2B} | n.s. | -- | n.s. | n.s. |
| h5-HT_{2C} | n.s. | -- | n.s. | n.s. |
| r5-HT₂ non-selective | n.s. | -- | -- | -- |
| h5-HT₃ | n.s. | -- | -- | -- |
| h5-HT₄ | -- | -- | n.s. | n.s. |
| gp5-HT₄ | n.s. | -- | -- | -- |
| h5-HT_{5A} | 98.5 | -- | -- | 63 |
| h5-HT₆ | n.s. | -- | n.s. | n.s. |
| h5-HT₇ | 0.6 | 0.6 | 31.6 | 1.25 |

| | | | | |
|---|---|---|---|---|
| n.s. not significant (K, > 1 µM or % inhibition at 1 µM lower than 50%); -- data not available. h, human, gp, guinea pig; m, mouse; r, rat; rb, rabbit, | | | | |

### Example 3: Sciatic Nerve ligation

### a) Effect of 5-HT₇ receptor ligands in neuropathic pain: von Frey test - mechanical allodynia

Partial sciatic nerve ligature induced mechanical allodynia. This was evidenced as a reduced pressure threshold for evoking withdrawal of the ipsilateral hind paw on day 10 post-injury compared to baseline pre-injury values (Fig. 16A). Systemic administration of the 5-HT₇ receptor agonist AS-19 on days 11-13 at doses of 1, 5 and 10 mg/kg significantly inhibited mechanical allodynia. At all these three doses. AS-19 restored thresholds of the nociceptive withdrawal response of nerve-injured paws to baseline pre-injury values, The analgesic (antiallodynic) effect disappeared as soon as AS-19 treatment was slopped (post-treatment thresholds the day after administration were indistinguishable from pre-treatment ones). Treatments with lower doses (0.1 and 0,3 mg/kg) of AS-19 were ineffective as no modification of this behavioral manifestation of neuropathic pain was found compared to vehicle-treated mice.
Sham operation did not induce mechanical allodynia but slight (not significant) reductions of the threshold of the nociceptive withdrawal response were usually found in sham-operated mice 10 days after surgery (Fig. 16B). AS-19 only produced minor effects in sham-operated mice although at doses of 1 and 5 mg/kg AS-19 increased the nociceptive threshold,
Activation of 5-HT₇ receptors by the selective agonist AS-19 has antiallodynic effects in nerve-injured mice, but does a selective 5-HT₇ receptor antagonist exert proallodynic effects? To investigate this possibility we administered the 5-HT, receptor antagonist SB-258719 to neuropathic mice, We observed that treatment with SB-258719 at 2.5 mg/kg significantly decreased the mechanical threshold for evoking withdrawal of the ipsilateral, nerve-injured hind paw below post-injury values (Fig. 17A). Interestingly, treatment with SB-258719 not only promoted allodynia in nerve-injured mice but it was also able to induce mechanical allodynia in sham-operated mice (Fig. 17B). In order to strengthen the notion that activation of 5-HT, receptors is responsible for the analgesic effect exerted by AS-19 we tried to pharmacologically reverse its antiallodynic effect with the antagonist SB-258719. As shown in Fig. 17B, the antiallodynic effect of the agonist and the proallodynic effect of the antagonist were inhibited when co-administered to neuropathic mice. In the same way, opposite effects of AS-19 (1 mg/kg) and SB-258719 (2.5 mg/kg) counteracted each other when administered together to sham-operated mice.

### b) Effect of 5-HT₇ receptor ligands in neuropathic pain: plantar test - thermal hyperalgesia

Partial sciatic nerve ligature induced thermal hyperalgesia as evidenced by decreased paw withdrawal latency to thermal stimulus on day 10 post-injury compared to baseline pre-injury values (Fig. 18A). However, a significant decrease of such a hyperalgesia was observed on days 11-13 post-injury in mice receiving treatment with the 5-HT₇ receptor agonist AS-19. In fact, treatment with AS-19 at doses of 1 and 10 mg/kg increased the withdrawal latency of nerve-injured paws to levels indistinguishable from control baseline pre-injury values, Lower doses (0.1 mg/kg) of AS-19 did not exert analgesic (antihyperalgesic) effect. Sham operation did not produce any significant modification of the nociceptive response and administration of AS-19 to sham-operated mice had no effects (Fig. 18B).
Similar to mechanical allodynia, thermal hyperalgesia was to some extend promoted by the 5-HT₇ receptor antagonist SB-258719 (Fig. 22). The decrease of paw withdrawal latency observed after administration of SB-258719 (2.5 mg/kg) in mice exposed to sciatic nerve injury did not reach statistical significance when compared to the vehicle-treated group. However, treatment with SB-258719 exerted a significant hyperalgesic effect and precipitated the appearance of thermal hyperalgesia in sham-operated mice. Finally, pharmacological reversion of the analgesic effect of AS-19 was accomplished by co-administering it with SB-258719 both in nerve-injured (Fig. 19A) and sham-operated mice (Fig. 19B),

### CONCLUSIONS

**5-HT₇ receptors play a key role in the control of neuropathic pain:**
- **Activation of 5HT₇ receptors exerts an analgesic effect in neuropathic pain: the 5HT₇ receptor agonist AS-19 inhibits neuropathic pain-associated behaviors (mechanical allodynia and thermal hyperalgesia).**
   The antiallodynic and antihyperalgesic effects were fully achieved by using low doses (1 mg/kg) of the selective 5HT₇ receptor agonist AS-19.
- **In contrast, blocking of 5HT₇ receptors promotes neuropathic pain: the 5HT₇, receptor antagonist SB-258719 exacerbates the behavioral manifestations of neuropathic pain (mechanical allodynia and thermal hyperalgesia).**
   The selective 5HT₇ receptor antagonist SB-258719 enhances allodynia and hyperalgesia in neuropathic pain conditions after nerve injury. In addition. SB-258719 precipitated the appearance behavioral manifestations of neuropathic pain in sham-operated mice.
- **Analgesic effect by the 5HT₇ receptor agonist and algesic effect by the 5HT₇ receptor antagonist can be pharmacologically reversed by co-administering both compounds**

**These results reveal the involvement of the 5-HT₇ receptor in the control of neuropathic pain and point to a new potential therapeutic use of 5-HT₇ receptor agonists as analgesics.**

### Example 4: Sciatic Nerve ligation

In another experiment using the technique of sciatic nerve ligation a dear antinociceptive effect of AS-19 was detected (Fig. 20).
Direct 5-HT7 receptor stimulation by AS-19 exerts long lasting anti-hyperalgesic/anti-allodynic-like effects in sciatic nerve-ligatured rats

### Example 5: Neuropathic Pain - Rats operated unilaterally on the infraorbital nerve

After pentobarbital-induced anaesthesia, the head of the rat was fixed in a stereotaxic frame, and a mid-line scalp incision was made, exposing skull and nasal bone. The infraorbital part of the right infraorbital nerve was then exposed. The edge of the orbit, formed by the maxillary, frontal, lacrimal and zygomatic bones, was dissected free, and orbital contents were gently deflected to give access to the infraorbital nerve. The latter was then dissected free at its most rostral extent in the orbital cavity, just caudal to the infraorbital foreamen. Two chromic catgut (5-0) ligatures were tied loosely (with about 2 mm spacing) around the nerve (Vos B.P., Strassman A.M. and Maciewitz R.J. (1994) Behavioural evidence of trigeminal neuropathic pain following chronic constriction injury to rat's infraorbital nerve. *J. Neurosci.* **14**: 2708-2723; and Kayser V., Aubel B., Hamon M. and Bourgoin S. (2002) The antimigraine 5-HT_{1B/1D} receptor agonists, sumatriptan, zolmitriptan and dihydroergotamine, attenuate pain-related behaviour in a rat model of trigeminal neuropathic pain. *Br. J. Pharmacol.* **137**: 1287-1297). Care was taken to avoid any interruption of the epineurial circulation. In sham-operated rats, the right infraorbital nerve was exposed, but it was not ligatured.

In these rats with unilateral ligatures on the infraorbital nerve, sensitivity to mechanical stimulation in the ipsilateral vibrissal territory was assessed by determination of pressure threshold, delivered through von Frey filaments (Semmes-Weinstein monofilaments, Stoelting, Wood Date. IL, USA), necessary to trigger defensive behavioural response (brisk withdrawal of the head; attack; escape reaction), For each stimulus filament (corresponding to a calibrated pressure of 0.217, 0.445, 0.745, 0.976, 2.35, 4.19, 6.00, 7.37 or 12.5 g), three consecutive applications (1 sec apart) were made in order to verify the stability of the response. In both sciatic nerve- and infraorbital nerve- ligatured rats, threshold values were determined first two days before surgery, then 14 days later, at a time when hyperesponsiveness to mechanical and thermal stimulations has fully developed (Vos et al., 1994).

Following the operation the response threshold in (g) is reduced considerably if in the operated rats and saline produced no modification of the nociceptive threshold (Fig. 21). AS-19 on the other hand clearly increased the threshold (Fig. 21).

AS-19 is quite potent and very effective to reduce mechanical allodynia in infraorbital nerve-ligatured rats.

5-HT7 receptors play a key role in the control of neuropathic pain in rats.

### Example 6: Carageenan induced acute and chronic inflammation

**a) Mechanical allodynia (measured by von_Frey filaments)**
   As can be seen by Figure 22, AS-19 has an antiallodynic effect in acute inflammation.
**b) Thermal hyperalgesia (measured by plantar test)**
   As can be seen by Figure 23. AS-19 has an antihyperalgesic effect in acute and especially chronic inflammation.

### Example 7: Streptozotocin model of diabetes induced neuropathic pain.

In a Streptozotocin model of diabetes induced neuropathic pain AS-19 seemed to have shown a dose-dependent antinociceptie effect using von-Frey filaments (see above).

## Claims

1. Use of a compound binding to the 5HT7 receptor and acting as a full or partial agonist in the production of a medicament for the treatment of pain.

2. Use, according to Claim 1, in which the pain is neuropathic pain, especially hyperalgesia or allodynia.

3. Use, according to claim 2, **characterized in that** the stimulus evoking the neuropathic pain is mechanical.

4. Use, according to claim 2, **characterized in that** the stimulus evoking the neuropathic pain is thermal.

5. Use, according to claim 2, **characterized in that** the neuropathic pain, is selected from central pain, hyperpathia, peripheral neuropathic pain or peripheral neurogenic pain, causalgia, hyperesthesia, neuralgia, neuritis, or neuropathy,

6. Use, according to any of claims 1 to 5, **characterized in that** the compound binding to the 5-HT7 receptor is binding with a higher affinity - expressed as a Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

7. Use, according to any of claims 1 to 5, **characterized in that** the compound binding to the 5-HT7 receptor is binding with a higher affinity - expressed as a Ki-value - to the 5HT7 receptor than to the any other 5-HT receptor; especially binding with an affinity higher by a factor of at least 10, preferably with an affinity higher by a factor of at least 30, more preferably with an affinity higher by a factor of at least 50, most preferably with an affinity higher by a factor of at least 100.

8. Use, according to any of claims 1 to 5, **characterized in that** the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM.

9. Use, according to any of claims 1 to 8, **characterized in that** the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM. most preferably lower than 10 nM
and
is binding with a higher affinity - expressed as a Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

10. Use according to any of claims 1 to 8, **characterized in that** the compound binding to the 5-HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a Ki-value - to the 5HT7 receptor than to any other 5HT receptor: especially binding with an affinity higher by a factor of at least 10, preferably with an affinity higher by a factor of at least 30, more preferably with an affinity higher by a factor of at least 50, most preferably with an affinity higher by a factor of at least 100.

11. Use according to any of claims 1 to 10, **characterized in that** the compound binding to the 5-HT7 receptor is acting as a full or partial agonist, especially a full agonist.

12. Use, according to any of claims 1 to 18, **characterized in that** the compound binding to the 5-HT7 receptor is AS-19 or MSD5a, especially AS-19, optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate.
